# DEMANDE DE BREVET EUROPEEN

(11) **EP 1 640 368 A2**
(43) Date de publication de la demande: **29.03.2006**
(21) Numéro de dépôt: 05015015.0
(22) Date de dépôt: 13.06.2001
(51) Int. Cl.: C07D 255/02, C07D 487/04, C07D 255/04, C07D 259/00, C07D 487/14, C07D 243/04, C07D 239/10, C07D 255/00, C07D 209/00

(54) **Composés urées cycliques et leur procédé de préparation**

(62) Demande divisionnaire de: 01945420.6
(71) Demandeur: The Centre National de la Recherche Scientifique, 75794 Paris Cedex 16 (FR); NEOMPS, 67100 Strasbourg (FR)
(72) Inventeur: Guichard, Gilles, 67202 Wolfisheim (FR); Plaue, Serge, 67500 Marienthal (FR); Semetey, Vincent, F-72000 Le Mans (FR); Schaffner, Arnaud-Pierre, 67150 Gerstheim (FR); Briand, Jean-Paul, 67000 Strasbourg (FR); Rodriguez, Marc, deceased (FR)
(74) Mandataire: Grosset-Fournier, Chantal Catherine

(57) **Abrégé**

La présente invention a pour objet un procédé de préparation de composés urées cycliques à partir d'au moins un dérivé activé d'acide carbamique contenant une fonction amine primaire ou secondaire non protégée, comprenant une étape de cyclisation par réaction entre la fonction amine primaire ou secondaire et la fonction acide carbamique dudit ou desdits dérivés d'acide carbamique.

## Description

La présente invention a pour objet un nouveau procédé de préparation de composés urées cycliques et de nouveaux composés urées cycliques.

La synthèse et les applications des urées substituées connaissent depuis quelques années un essor important. En particulier, les urées cycliques sont présentes dans un certain nombre de principes actifs actuellement en développement dans l'industrie pharmaceutique comme des inhibiteurs de la protéase du VIH, ou bien des inhibiteurs du facteur Xa (fXa). Les urées cycliques décrites dans la littérature (WO 93/07128, WO 96/29329, WO 97/08150, WO 98/20009) possèdent généralement des cycles à 5, 6, 7 ou 8 atomes. Ces cycles peuvent parfois contenir un hétéro atome supplémentaire, tel qu'un atome d'azote adjacent à la fonction urée (Sham et al., Journal of Medicinal Chemistry, 1996, 39, 392-397). Le cycle urée des urées cycliques biologiquement actives décrites dans la littérature, est utilisé comme une plate-forme de conformation restreinte sur laquelle sont disposés les groupements pharmacophores servant à la reconnaissance par la protéase du VIH. Il est donc important de disposer d'une méthode de synthèse suffisamment flexible pour permettre facilement l'introduction d'une diversité moléculaire au niveau des groupements pharmacophores ainsi qu'au niveau de la position de ces groupements sur le cycle. Les urées cycliques décrites dans la littérature sont généralement préparées à partir des diamines correspondantes par cyclisation intramoléculaire desdites diamines à l'aide d'un agent de carbonylation comme le carbonyldiimidazole. L'alkylation d'urées N,N'-disubstituées par des bis-halogénure d'alkyle pour la préparation d'urées cycliques a également été décrite (WO96/00708 et WO 93/07128).

Dans le cadre de recherches visant à développer de nouveaux composés à activité immunomodulatrice, la Société demanderesse a précédemment développé une méthode simple et efficace permettant la préparation de nouveaux dérivés activés stables d'acide carbamique, à partir d'un dérivé d'acide aminé N-protégé, comprenant trois étapes :
a) une étape de transformation du groupe -COOH du dérivé d'acide aminé N-protégé (acides aminés α, β, γ et δ) en groupe -CON₃ pour obtenir un acyl azide,
b) une étape de transformation du groupe -CON₃ de l'acyl azide en groupe -NCO pour obtenir un isocyanate,
c) une étape de traitement de l'isocyanate pour obtenir le susdit dérive stable d'acide carbamique.

Le terme "dérivé d'acide aminé" doit s'interpréter au sens large, tel que compris par l'homme du métier, et désigne notamment un dérivé de peptide, de polypeptide, de protéine, de pseudopeptide ou d'oligourée.

Les dérivés d'acide carbamique sont des intermédiaires stables et cristallins, qui réagissent avec des amines pour former des urées substituées. Les dérivés d'acide carbamique permettent également de préparer des peptides contenant des motifs urées (« Guichard et al., J. Org. Chem. 1999, 64, 8702-8705 », et « Guichard et al., Tetrahedron Letter, 2000, 41, 1553-1557 »).

L'un des aspects de l'invention est de proposer un nouveau procédé de préparation de composés urées cycliques.

L'un des autres aspects de l'invention est de proposer un nouveau procédé de préparation de composés urées cycliques, permettant d'obtenir facilement et en très peu d'étapes une grande diversité moléculaire de composés urées cycliques.

L'un des autres aspects de l'invention est de proposer de nouveaux composés urées cycliques.

Dans sa généralité, l'invention a pour objet un procédé de préparation de composés urées cycliques à partir d'au moins un dérivé activé d'acide carbamique contenant une fonction aminé primaire ou secondaire non protégée, comprenant une étape de cyclisation par réaction entre la fonction amine primaire ou secondaire et la fonction acide carbamique dudit ou desdits dérivés d'acide carbamique.

Par "dérivé activé d'acide carbamique", on désigne un dérivé d'acide carbamique, notamment un carbamate, contenant une fonction amine primaire ou secondaire capable de réagir avec des amines primaires ou secondaires en présence ou non d'une base dans un solvant organique.

Par fonction amine primaire ou secondaire "non protégée", on entend une fonction amine primaire ou secondaire libre, c'est-à-dire capable de réagir avec un autre groupement fonctionnel chimique, et notamment avec une fonction acide carbamique. La fonction amine primaire ou secondaire "non protégée" pourra encore être dénommée dans ce qui suit, fonction amine primaire ou secondaire "libérée", "libre" ou "déprotégés".

Dans ce qui suit, on entend encore par "dérivé activé d'acide carbamique contenant une fonction amine primaire ou secondaire non protégée" :
- un dérivé d'acide carbamique oligomérique, encore dénommé dans ce qui suit "dérivé activé d'acide carbamique homo-oligomérique", ou "dérivé homo-oligomère" ou "dérivé activé d'acide carbamique hétéro-oligomérique" ou "dérivé hétéro-oligomère" ou,
- un dérivé d'acide carbamique monomérique, correspondant à un dérivé activé d'acide carbamique non homo-oligomérique ou à un dérivé activé d'acide carbamique non hétéro-oligomérique.

Le ou les dérivés activés d'acide carbamique homo-oligomériques et/ou hétéro-oligomériques sont obtenus à l'issue d'une ou plusieurs réactions d'homo-oligomérisations et/ou d'hétéro-oligomérisations d'un moins un dérivé activé d'acide carbamique monomérique. Les réactions d'homo-oligomérisation ou d'hétéro-oligomérisation peuvent encore être dénommées dans ce qui suit "réactions intermoléculaires".

Par "étape de cyclisation", il faut entendre une étape de cyclisation intramoléculaire ou, une étape de cyclisation intermoléculaire.

Une cyclisation intramoléculaire a lieu par réaction entre la fonction amine primaire ou secondaire non protégée du dérivé activé d'acide carbamique et sa fonction acide carbamique.

Une cyclisation intermoléculaire a lieu par réaction entre :
- la fonction amine primaire ou secondaire non protégée d'un dérivé activé d'acide carbamique (dénommé dérivé 1) et la fonction acide carbamique d'un autre dérivé activé d'acide carbamique (dénommé dérivé 2) et,
- la fonction amine primaire ou secondaire non protégée dudit dérivé 2 d'acide carbamique et la fonction acide carbamique dudit dérivé 1 d'acide carbamique.

Selon un mode de réalisation avantageux, l'invention concerne un procédé de préparation de composés urées cycliques comprenant:
- une étape d'obtention d'au moins un dérivé activé d'acide carbamique contenant une fonction amine primaire ou secondaire non protégée, à partir d'au moins un dérivé activé stable d'acide carbamique contenant une fonction amine protégée par un groupe protecteur, par libération sélective de ladite fonction amine protégée dudit ou desdits dérivés activé(s) stable(s) d'acide carbamique, par clivage ou transformation dudit groupe protecteur,
- une étape de cyclisation par réaction entre la fonction amine primaire ou secondaire non protégée d'au moins un dérivé activé obtenu à l'issue de l'étape de libération sélective et la fonction acide carbamique du ou des dérivés.

Selon un mode de réalisation avantageux du procédé de l'invention, l'étape de cyclisation est une cyclisation intramoléculaire entre la fonction amine primaire ou secondaire non protégée d'un dérivé activé obtenu à l'issue de l'étape de libération sélective et sa fonction acide carbamique.

On entend par "groupe protecteur", un groupe protégeant la fonction amine du dérivé activé d'acide carbamique, afin notamment de l'empêcher de réagir avec d'autres groupements fonctionnels chimiques, lors de la synthèse dudit dérivé.

Par "dérivé activé stable d'acide carbamique" on désigne un dérivé d'acide carbamique isolable, purifiable et stockable (de préférence à 4°C) pour une période d'au moins 3 mois sans dégradation notable. La stabilité peut être mesurée par exemple par le test suivant : chromatographie liquide haute performance (CLHP), chromatographie sur couche mince (CCM), résonance magnétique nucléaire (RMN) ou infra rouge (IR).

Par "libération sélective" de la fonction amine protégée, il faut comprendre une libération permettant de libérer uniquement la fonction amine protégée du dérivé activé stable d'acide carbamique sans altérer la fonction acide carbamique dudit dérivé. Dans ce qui suit, l'étape de "libération sélective" peut encore être dénommée "déprotection sélective".

L'étape de libération ou de déprotection sélective de la fonction amine protégée par un groupe protecteur est dépendante :
- du groupement protecteur utilisé pour protéger la fonction amine et,
- du réactif utilisé lors de la déprotection ou libération de la fonction amine.

La libération d'une fonction amine par clivage du groupement protecteur ou par transformation du groupement protecteur est effectuée selon les méthodes classiques décrites dans la littérature.

Selon un mode de réalisation avantageux du procédé de l'invention, lé dérivé activé stable d'acide carbamique contenant une fonction amine protégée par un groupe protecteur est obtenu à partir d'un dérivé d'acide aminé dans lequel le groupe aminé est protégé, par un procédé tel que décrit ci-dessus et dont les trois étapes sont les suivantes :
- transformation du groupe -COOH du dérivé d'acide aminé N-protégé en groupe -CON₃ pour obtenir un acyl azide,
- transformation du groupe -CON₃ de l'acyl azide en groupe -NCO pour-obtenir un isocyanate,
- traitement de l'isocyanate -NCO pour obtenir le susdit dérivé stable d'acide carbamique.

Selon un mode de réalisation avantageux du procédé de préparation de l'invention, la fonction amine primaire ou secondaire non protégée du dérivé activé d'acide carbamique se présente :
(1) sous forme libre et/ou,
(2) sous forme protonée, notamment sous forme de sel.

A titre d'exemple d'un dérivé activé d'acide carbamique contenant une fonction amine primaire ou secondaire sous forme protonée, notamment sous forme de scl, on peut notamment citer un sel d'acétate, un sel de chlorhydrate ou un sel de trifluoroacétate.

Le dérivé activé d'acide carbamique contenant une fonction amine primaire ou secondaire non protégée sous forme protonée peut être isolé, tandis que le dérivé activé d'acide carbamique contenant une fonction amine primaire ou secondaire non protégée sous forme libre ne peut pas être isolé : dans ce dernier cas, l'étape de cyclisation a lieu immédiatement après l'obtention d'au moins un dérivé activé d'acide carbamique contenant une fonction amine primaire ou secondaire non protégée.

Selon un mode de réalisation avantageux de l'invention, le procédé de préparation des composés urées cycliques comprend, au cours ou à l'issue de l'étape de libération sélective, une étape d'homo-oligomérisation et/ou d'hétéro-oligomérisation entre :
- la fonction amine primaire ou secondaire non protégée d'une molécule du dérivé activé d'acide carbamique et la fonction acide carbamique d'une autre molécule dudit dérivé activé d'acide carbamique et/ou,
- entre la fonction amine primaire ou secondaire non protégée d'une molécule du dérivé activé d'acide carbamique et la fonction acide carbamique d'une molécule d'un autre dérivé activé d'acide carbamique, pour obtenir au moins un dérivé homo-oligomérique et/ou hétéro-oligomérique d'acide carbamique contenant une fonction amine primaire ou secondaire non protégée.

Ainsi, selon un mode de réalisation avantageux, le procédé de préparation de l'invention comprend, au cours ou à l'issue de l'étape de libération sélective :
- une étape d'homo-oligomérisation entre la fonction amine primaire ou secondaire non protégée d'une molécule du dérivé activé d'acide carbamique et la fonction acide carbamique d'une autre molécule dudit dérivé activé d'acide carbamique, pour obtenir au moins un dérivé homo-oligomérique d'acide carbamique contenant une fonction amine primaire ou secondaire non protégée ou,
- au moins une étape d'homo-oligomérisation entre la fonction amine primaire ou secondaire non protégée d'une molécule d'un dérivé activé d'acide carbamique et la fonction acide carbamique d'une autre molécule dudit dérivé activé d'acide carbamique, et au moins une étape d'hétéro-oligomérisation entre la fonction amine primaire ou secondaire non protégée d'une molécule d'un dérivé activé d'acide carbamique et la fonction acide carbamique d'une molécule d'un autre dérivé activé d'acide carbamique, pour obtenir au moins un dérivé homo-oligomérique et au moins un dérivé hétéro-oligomérique d'acide carbamique contenant une fonction amine primaire ou secondaire non protégée.

Ainsi, les précurseurs acycliques bifonctionnels obtenus au cours ou à l'issue de la libération sélective de la fonction amine protégée, à savoir les dérivés activés monomériques d'acide carbamique contenant une fonction amine primaire ou secondaire sous forme libre ou prolonée, peuvent subir, avant l'étape de cyclisation intramoléculaire, des réactions intermoléculaires d'homo- et/ou d'hétéro-oligomérisation afin de former des précurseurs acycliques bifonctionnels homo-oligomériques et/ou hétéro-oligornériques. Les dérivés acycliques d'acide carbamique homo-oligomériques et/ou hétéro-oligomériques ainsi obtenus subissent ensuite, tout comme les dérivés acycliques d'acide carbamique non homo- et/ou non hétéro-oligomériques, une cyclisation (ou macrocyclisation) intramoléculaire par réaction de leur fonction amine primaire ou secondaire non protégée avec leur fonction acide carbamique, afin d'obtenir des urées cycliques homo-oligomériques et/ou hétéro-oligomériques.

Cependant, pour que les réactions intermoléculaires d'homo-oligomérisation et/ou d'hétéro-oligomérisation, et les réactions de cyclisations intramoléculaires puissent avoir lieu, la fonction amine primaire ou secondaire libérée du dérivé activé d'acide carbamique doit être sous forme libre et non pas sous forme protonée. En effet, seule la fonction amine primaire ou secondaire sous forme libre peut réagir par réaction intermoléculaire d'homo- ou d'hétéro-oligomérisation, ou par cyclisation intramoléculaire.

Ainsi, lorsque le dérivé activé d'acide carbamique, obtenu à l'issue de l'étape de libération sélective, contient une fonction amine primaire ou secondaire non protégée sous forme libre, les réactions d'homo- et/ou d'hétéro-oligomérisation, et de cyclisation intramoléculaire peuvent avoir lieu immédiatement après la formation du dérivé activé d'acide carbamique contenant une fonction amine primaire ou secondaire non protégée sous forme libre, car ladite fonction amine sous forme libre peut réagir avec le groupe acide carbamique activé.

Lorsque le dérivé activé d'acide carbamique, obtenu à l'issue de l'étape de libération sélective, contient une fonction amine primaire ou secondaire non protégée sous forme protonée, il sera nécessaire de neutraliser au préalable ladite forme protonée de l'amine en forme libre afin que les réactions intermoléculaires d'homo-oligomérisation et/ou d'hétéro-oligomérisation, et les réactions de cyclisations intramoléculaires puissent avoir lieu.

Selon un mode de réalisation avantageux du procédé de préparation des composés urées cycliques, lorsque le dérivé activé d'acide carbamique contient une fonction amine primaire ou secondaire sous forme protonée, l'étape d'homo-oligomérisation et/ou d'hétéro-oligomérisation est effectuée en neutralisant la fonction amine primaire ou secondaire sous forme protonée en fonction amine primaire ou secondaire sous forme libre, pour obtenir au moins un dérivé homo-oligomérique et/ou hétéro-oligomérique contenant une fonction aminé primaire ou secondaire non protégée sous forme libre.

Selon un autre mode de réalisation avantageux du procédé de préparation des composés urées cycliques, lorsque le dérivé activé d'acide carbamique contient une fonction amine primaire ou secondaire sous forme protonée, l'étape de cyclisation est effectuée en neutralisant la fonction amine primaire ou secondaire sous forme protonée en fonction amine primaire ou secondaire sous forme libre.

La neutralisation de ladite fonction aminé protonée en forme libre est notamment effectuée à l'aide d'une base choisie dans le groupe constitué par la diisopropyléthylamine, la triéthylamine, la lutidine, la pyridine, la 2,4,6-collidine, la N-méthylmorpholine, la 2,6-di-tert-butyl-4-méthylpyridine ou leurs mélanges.

Par ailleurs, afin d'effectuer l'étape de cyclisation intramoléculaire des dérivés activés d'acide carbamique oligomériqucs ou monomériques contenant une fonction amine primaire ou secondaire, on pourra notamment utiliser un solvant choisi dans le groupe constitué par l'acétonitrile (MeCN), le toluène, la pyridine, le N,N-diméthylformamide (DMF), le tétrahydrofurane (THF), le chloroforme, le dichlorométhane, la N-méthylpyrrolidone (NMP), le diméthylsulfoxide (DMSO), l'acétate d'éthyle, le méthanol, l'éthanol ou leurs mélanges.

Le solvant utilisé pour effectuer l'étape de cyclisation est dénommé dans ce qui suit "solvant de réaction" ou "solvant de cyclisation".

Ainsi, l'étape de cyclisation intramoléculaire de dérivés activés d'acide carbamique contenant une fonction aminé primaire ou secondaire non protégée sous forme protonée est effectuée en présence :
- d'une base afin de neutraliser la forme protonée de l'amine en forme libre et,
- du solvant de réaction pour la cyclisation intramoléculaire,
tandis que l'étape de cyclisation intramoléculaire de dérivés activés d'acide carbamique contenant une fonction amine primaire ou secondaire non protégée sous forme libre est effectuée directement à l'aide d'un solvant de réaction pour la cyclisation intramoléculaire.

Selon un mode de réalisation avantageux du procédé de l'invention, l'étape de cyclisation intramoléculaire du dérivé activé d'acide carbamique contenant une fonction aminé primaire ou secondaire non protégée est effectuée à une température d'environ -40°C à environ 40°C, notamment d'environ -20°C à environ 40°C, et de préférence d'environ 0°C à environ 20°C.

Selon un autre mode de réalisation avantageux du procédé de l'invention, la concentration d'un dérivé activé d'acide carbamique contenant une fonction amine primaire ou secondaire non protégée sous forme libre, dans une solution contenant un solvant de réaction pour la cyclisation intramoléculaire, est d'environ 10⁻⁶ M à environ 10 M, notamment d'environ 10⁻⁵ M à environ 1 M, et de préférence d'environ 10⁻⁴ M à environ 1 M.

Selon un autre mode de réalisation avantageux du procédé de l'invention, la concentration d'un dérivé activé d'acide carbamique contenant une fonction amine primaire ou secondaire non protégée sous forme protonée, dans une solution contenant un solvant de réaction pour la cyclisation intramoléculaire et une base, est d'environ 10⁻⁶ M à environ 10 M, notamment d'environ 10⁻⁵ M à environ 1 M, et de préférence d'environ 10⁻⁴ M à environ 1 M.

La concentration de la base dans le solvant de réaction pour la cyclisation intramoléculaire, est d'environ 10⁻⁶ M à environ 10 M, notamment d'environ 10⁻⁵ M à environ 1 M, et de préférence d'environ 10⁻⁴ M à environ 1 M.

Selon un mode de réalisation avantageux du procédé de préparation des composés urées cycliques, le dérivé activé d'acide carbamique contenant une fonction amine protégée est synthétisé sur support solide, et est lié chimiquement audit support solide soit (a) par sa fonction amine, soit (b) par sa fonction acide carbamique, soit (c) par tout autre groupement fonctionnel présent dans ledit dérivé activé d'acide carbamique.

Par "support solide", il faut comprendre la matrice sur laquelle la réaction chimique est effectuée. Il s'agit en général d'un polymère solide insoluble qui permet la filtration ou la centrifugation, et donc la séparation des réactifs et du produit formé sur la résine. A titre d'exemple de support solide, on pourra citer les résines polystyréniques, le polyacrylamide, le polyéthylèneglycol, la cellulose, le verre, la silice.

Selon un mode de réalisation avantageux du procédé de préparation de l'invention, lorsque le dérivé activé d'acide carbamique contenant une fonction amine protégée est lié chimiquement à un support solide :
- par sa fonction aminé, l'étape de libération sélective entraîne le clivage de la fonction amine dudit dérivé par rapport au support,
- par sa fonction acide carbamique, l'étape de cyclisation entraîne le clivage de la fonction acide carbamique dudit dérivé par rapport au support,
- par un groupement fonctionnel autre que la fonction amine ou acide carbamique tel qu'une fonction hydroxyle, une fonction amide ou une fonction carboxyle, le clivage dudit groupement fonctionnel par rapport au support peut avoir lieu au cours ou à l'issue de l'une quelconque des étapes de libération sélective ou de cyclisation.

Selon un autre mode de réalisation avantageux du procédé de préparation l'invention, la fonction amine du dérivé activé d'acide carbamique est protégée sous la forme d'un groupement :
- carbamate (ROCON-) dans lequel R est un groupe tert-butyle, 9-fluorénylméthyle, benzyle, allyle, tert-butyldiméthylsilyle, éthyle, 2,2,2-trichloroéthyle, 2-(triméthylsilyl)éthyle,
- amine tertiaire de formule R'N< lorsque la fonction amine à protéger est une amine secondaire, ou de formule R'R"N- lorsque la fonction amine à protéger est une amine primaire, R' et R" représentant chacun un groupe protecteur choisi dans le groupe constitué par le benzyle, le 4-méthoxybenzyle, le 2,4-diméthoxybenzyle, le diphenylméthyle, le para-méthoxyphenyle, le 3,4-diméthoxybenzyle ou le 9-phenyl-9-fluorényle,
- amide,
- nitro,
- azide,
- trityle,
- ortho-(ou para)-nitrophénylsulfonyle,
- tosyle,
- phtalimide, ou
- cyano.

La libération de la fonction amine par clivage du groupement protecteur est effectuée selon les méthodes classiques décrites dans la littérature. A cet égard, on peut citer l'ouvrage intitulé "Protecting groups" de P.J. Kocienski (édition Thieme), qui donne une liste exhaustive des groupements protecteurs des fonctions amines et de leurs modes de déprotection.

A titre d'exemple, on peut notamment citer :
- la déprotection du groupement tert-butoxycarbonyle (ROCO- avec R = groupe tert-butyle) (encore appelé groupement Boc) en condition acide (acide trifluoroacétique ou solution d'acide chlorhydrique en solvant organique à 3-4 M) qui conduit à l'obtention de l'amine correspondante sous forme de sel de trifluoroacétate ou de chlorhydrate,
- la déprotection du groupement benzyloxycarbonyle (ROCO- avec R = groupe benzyle) (encore appelé groupement Z) ou des amines tertiaires (de formule R'N< ou R'R"N-) contenant un ou deux groupes benzyle, par hydrogénatrion catalytique en présence de Pd/C, avec ajout ou non d'un acide pour protoner ou non l'amine primaire ou secondaire libérée.

La libération de la fonction amine par transformation d'un groupement chimique servant de forme cachée de l'amine tel qu'un groupement nitro, cyano, amide ou azide est notamment décrite dans les exemples ci-dessous. Cependant, ces exemples ne sont pas limitatifs car il existe un grand nombre de méthodes permettant de réaliser les transformations décrites ci-dessous ("Textbook of Practical Organic Chemistry" de Vogel, (5^{ème} édition), 1989).

La libération de la fonction amine peut être effectuée par réduction des groupes nitro ou cyano en amine, par exemple par hydrogénation catalytique en présence de Pd/C et PtO₂ respectivement.

La transformation d'un groupement amide en groupe amine peut être effectuée par réarrangement d'Hoffman, par exemple par traitement de l'amide avec de l'iodobenzyl bis-trifluoroacétate dans un mélange cau/acétonitrile.

La réduction d'un groupement azide en groupe amine peut être effectuée par différentes méthodes, par exemple par hydrogénation catalytique ou par traitement avec de l'hydrure de lithium aluminium.

L'invention a également pour objet des composés urées cycliques comportant un cycle ayant au moins 7 atomes, notamment de 7 à 50 atomes, et de préférence de 7 à 20 atomes, lequel cycle comprenant au moins une fonction amide et au moins une fonction urée, chaque fonction amide ou urée étant séparée de la plus proche fonction adjacente amide ou urée par au moins un atome de carbone, et notamment par 1 à 4 atomes de carbone.

L'invention a notamment pour objet des composés urées cycliques comportant un cycle ayant au moins 7 atomes, notamment de 7 à 50 atomes, et de préférence de 7 à 10 atomes, lequel cycle comprenant une fonction amide et une fonction urée séparées l'une de l'autre par au moins un atome de carbone, et notamment par 1 à 4 atomes de carbone.

A cet égard, l'invention concerne des composés urées cycliques de formule (Ia) : 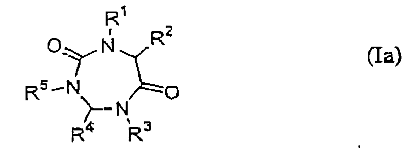 dans laquelle les groupes R¹, R², R³, R⁴ et R⁵ peuvent représenter chacun et indépendamment les uns des autres :
a) - un hydrogène,
b) - un halogène,
c) - la chaîne latérale protégée ou non d'un acide aminé choisi parmi les acides aminés naturels ou non naturels,
d) - un groupe alkyle (C1-C20), linéaire ou ramifié, non substitué ou substitué par un ou plusieurs substituants parmi lesquels : -COORₐ, -CONHRₐ, -ORₐ, -NHRₐ, -NH(CO)Rₐ, -NHCOORₐ, un groupement aryle ou hétéroaryle, dont la structure cyclique contient de 5 à 20 atomes de carbone, un atome d'halogène, un groupement R'''CO-, le groupe R''' comprenant de 1 à 10 atomes de carbone, un groupe nitrile, guanidino ou nitro,
e) - un groupement aryle dont la structure du cycle contient de 5 à 20 atomes de carbone, substitué ou non par les substituants mentionnés ci-dessus, ainsi que par les groupements cyano ou amidine,
f) - un groupement alkényle ou alkynyle (C1-C6)
g) - un groupement sulfonyle (R_{c}SO2)
h) - un groupement acyle (R_{c}CO)
i) - un groupement OR_{b}
j) - un groupement NH₂
k) -COOR_{b}
1) -CONHR_{b}
m) -CH₂CONH₂
Rₐ et R_{b} représentant indépendamment l'un de l'autre un hydrogène, un groupe allyle, benzyle, t-butyle, fluorénylméthyle, benzyloxyméthyle, tert-butyldiméthylsilyle, 2-éthoxyéthyle, méthoxyméthyle, 2-méthoxyéthoxyméthyle, tétrahydropyran-2-yle, triméthylsilyle, triéthylsilyle, 2-(triméthylsilyl)éthyle, trityle, 2,2,2-trichloroéthyle, tosyle, ortho-(ou para)-nitrophénylsulfonyle, alkyle ayant de 1 à 20 atomes de carbone, ou un groupe aryle dont la structure du cycle contient de 5 à 20 atomes de carbone,
R_{c} représentant on groupement alkyle ayant de 1 à 20 atomes de carbone, ou un groupe aryle dont la structure du cycle contient de 5 à 20 atomes de carbone, ou un groupe hétéroaryle, arylalkyle ou hétéroarylalkyle,
les groupes R¹, R², R³ et R⁴ pouvant également former les cyclisations intramoléculaires suivantes :
1/ cyclisation entre R¹ et R² et/ou,
2/ cyclisation entre R³ et R⁴,
lesdits composés urées cycliques pouvant être, lorsqu'un ou plusieurs carbones asymétriques sont présents dans la formule (Ia), de façon indépendante, soit de configuration R (rectus) soit de configuration S (sinister).

Un groupe avantageux de composés urées cycliques répondant à la formule générale (la) est constitué par les composés urées cycliques répondant plus particulièrement aux formules (Ib), (Ic), (Id), (Te), (If), (Ig), (Ih) : 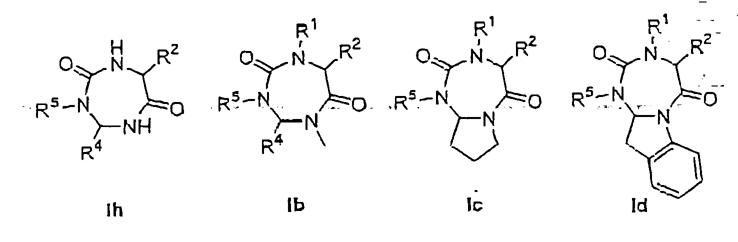 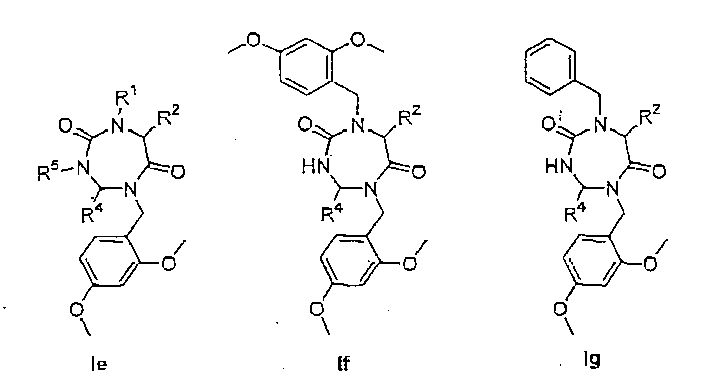 dans lesquelles les groupes R¹, R², R⁴ et R⁵ ont les significations indiquées ci-dessus,
les groupes R¹ et R² pouvant également former une cyclisation intramoléculaire,
lesdits composés urées cycliques pouvant être, lorsqu'un ou plusieurs carbones asymétriques sont présents dans les formules (Ib) à (Ih), de façon indépendante, soit de configuration R (rectus) soit de configuration S (sinister).

Dans les composés représentés ci-dessus et ci-après, la liaison « ― » représente un groupe méthyle, et pourrait également être représentée de la façon suivante : « ―CH₃ ».

L'invention concerne également les composés urées cycliques de formules (IIa), (IIb), (IIc), (IId) : 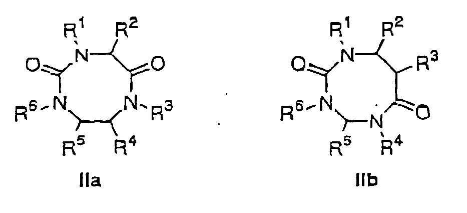 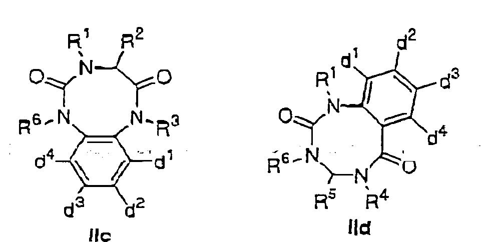 dans lesquelles les groupes R¹, R², R³, R⁴, R⁵ et R⁶ ont les significations indiquées ci-dessus à propos des groupes R¹ à R⁵,
les groupes R¹, R², R³, R⁴, R⁵ pouvant également former les cyclisations intramoléculaires suivantes :
1/ cyclisation entre R¹ et R² ou,
2/ cyclisation entre R² et R³ et/ou,
3/ cyclisation entre R⁴ et R⁵ ou,
4/ cyclisation entre R⁵ et R⁶,
les groupes d¹, d², d³ et d⁴ peuvent représenter chacun et indépendamment les uns des autres un groupement : nitro, alkyle comprenant de 1 à 4 atomes de carbone, et notamment un méthyle, alkoxy comprenant de 1 à 7 atomes de carbone, et notamment un méthoxy, aryloxy comprenant de 5 à 10 atomes de carbone, et notamment un benzyloxy, halogène tel qu'un fluoro, bromo, chloro ou iodo, CN, guanidino, NHRₐ, NHCOORₐ, COORₐ, ORₐ,
Rₐ ayant les significations indiquées ci-dessus,
lesdits composés urées cycliques pouvant être, lorsqu'un ou plusieurs carbones asymétriques sont présents dans les formules (IIa) à (IId), de façon indépendante, soit de configuration R (rectus) soit de configuration S (sinister).

L'invention concerne également les composés urées cycliques comportant un cycle ayant au moins 14 atomes, notamment de 14 à 30, de préférence 14 à 20, lequel cycle comprenant deux fonctions amide et deux fonctions urée, chaque fonction amide ou urée étant séparée de la plus proche fonction adjacente amide ou urée par au moins un atome de carbone, et notamment par 1 à 4 atomes de carbone.

A cet égard., l'invention concerne les composés urées cycliques de formule (IIIa): 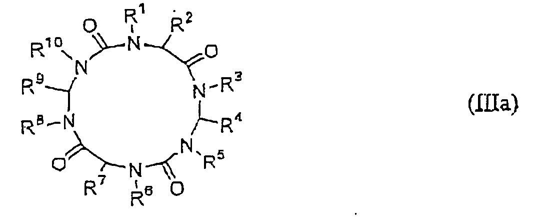 dans laquelle les groupes R¹, R², R³, R⁴, R⁵, R⁶, R⁷, R⁸, R⁹ et R¹⁰ ont les significations mentionnées ci-dessus à propos des groupes R¹ à R⁵,
les groupes R¹, R², R³, R⁴, R⁵, R⁶, R⁷, R⁸, R⁹ peuvent également former les cyclisations intramoléculaires suivantes :
1/ cyclisation entre R¹ et R² et/ou,
2/ cyclisation entre R³ et R⁴ et/ou,
3/ cyclisation entre R⁶ et R⁷ et/ou,
4/ cyclisation entre R⁸ et R⁹,
lesdits composés urées cycliques pouvant être, lorsqu'un ou plusieurs carbones asymétriques sont présents dans la formule (IIIa), de façon indépendante, soit de configuration R (rectus) soit de configuration S (sinister).

Un groupe avantageux de composés urées cycliques répondant à la formule générale (IIIa) est constitué par les composés urées cycliques répondant plus particulièrement aux formules (IIIb), (IIIc), (IIId), (IIIe), (IIIf) et (IIIg) : 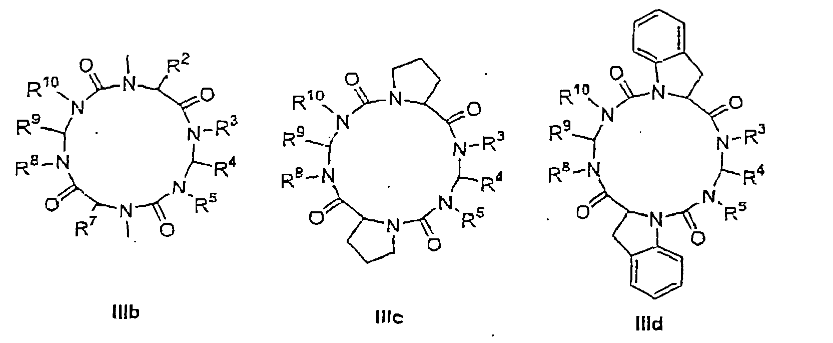 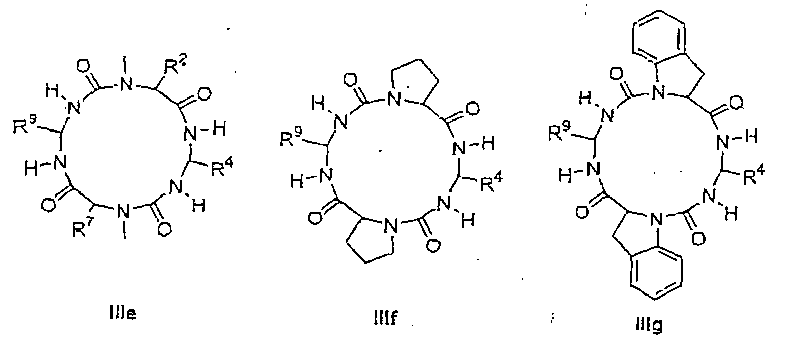 dans lesquelles les groupes R², R³, R⁴, R⁵, R⁷, R⁸, R⁹ et R¹⁰ ont les significations mentionnées ci-dessus à propos des groupes R¹ à R⁵,
les groupes R³, R⁴, R⁷, R⁸ et R⁹ pouvant également former les cyclisations intramoléculaires telles que définies ci-dessus à propos des groupes R¹, R², R³, R⁴, R⁵, R⁶, R⁷, R⁸, R⁹ des composés de formule générale (IIIa),
lesdits composés urées cycliques pouvant être, lorsqu'un ou plusieurs carbones asymétriques sont présents dans les formules (IIIb) à (IIIg), de façon indépendante, soit de configuration R (rectus) soit de configuration S (sinister).

L'invention a également pour objet des composés urées cycliques comportant un cycle ayant 7 atomes et comprenant une fonction urée, de formule (IVa) : 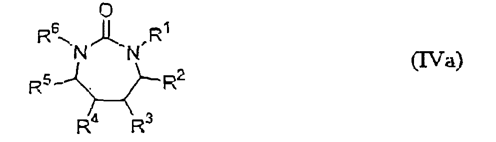 dans laquelle R¹, R², R³, R⁴, R⁵ et R⁶ ont les significations mentionnées ci-dessus à propos des groupes R¹ à R⁵,
avec la restriction que si R³ = R⁴ = OH alors R² doit être différent de R⁵,
les groupes R¹, R², R³, R⁴, R⁵ pouvant également former les cyclisations intramoléculaires suivantes :
1/ cyclisation entre R' et R² et/ou,
2/ cyclisation entre R² et R³ et/ou,
3/ cyclisation entre R³ et R⁴ et/ou,
4/ cyclisation entre R⁴ et R⁵,
lesdits composés urées cycliques pouvant être, lorsqu'un ou plusieurs carbones asymétriques sont présents dans la formule (IVa), de façon indépendante, soit de configuration R (rectus), soit de configuration S (sinister).

Selon un mode de réalisation avantageux, le dérivé activé d'acide carbamique contenant une fonction amine primaire ou secondaire non protégée impliqué dans le procédé de préparation de l'invention répond respectivement :
- soit à l'une des formules (VIa), (VIb), (VIc) ou (VId) suivantes (pour obtenir les composés de formules (Ia) à (Ib) tels que définis ci-dessus) : 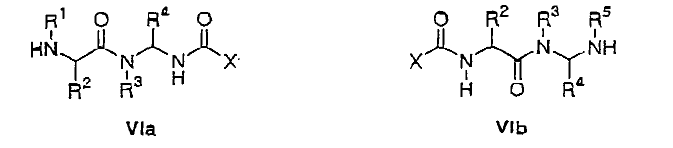 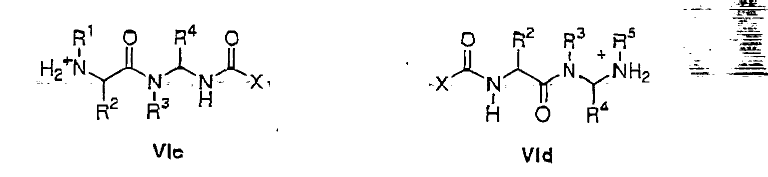 dans lesquelles
   le groupe X représente un groupe conférant au dérivé une structure de dérivé activé d'acide carbamique, lequel groupe X étant issu d'un composé choisi notamment parmi les phénols, éventuellement substitués par au moins un groupe nitro ou au moins un halogène, ou les dérivés d'hydroxylamine, ou les dérivés d'alcools benzyliques greffés sur support solide et plus particulièrement choisi parmi les composés suivants : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzotriazine (HODhbt), 7-aza-1-hydroxy-benzotriazole (HOAt), 4-aza-1-hydroxybenzotriazole (4-HOAt), imidazolc, tétrazole, résine WANG,
   les groupes R¹, R², R³ R⁴ et R⁵ ont les significations mentionnées ci-dessus à propos des groupes R¹ à R⁵,
- soit à l'une des formules (VIIa), (VIIb), (VIIc) ou (VIId) suivantes (pour obtenir les composés de formules (IIa) tels que définis ci-dessus) : 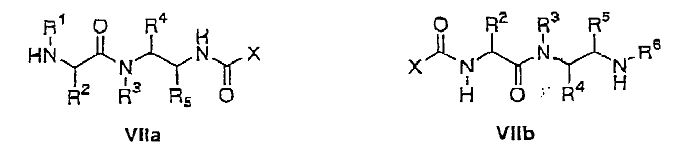 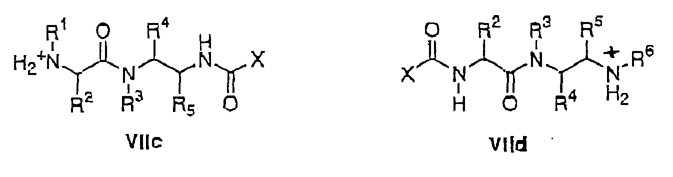 dans lesquelles
   X est tel que défini ci-dessus,
   les groupes R¹, R², R³, R⁴, R⁵ et R⁶ ont les significations mentionnées ci-dessus à propos dcs groupes R¹ à R⁵,
- soit à l'une des formules (VIIIa), (VIIIb), (VIIIc) ou (VIIId) suivantes (pour obtenir les composés de formules (IIb) tels que définis ci-dessus) : 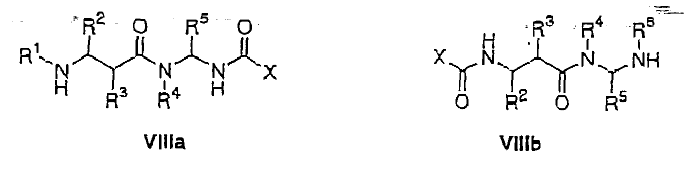 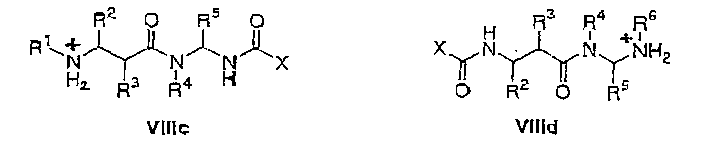 dans lesquelles
   X est tel que défini ci-dessus,
   les groupes R¹, R², R³, R⁴, R⁵ et R⁶ ont les significations mentionnées ci-dessus à propos des groupes R¹ à R⁵,
- soit à l'une des formules (IXa), (IXb), (IXc) ou (IXd) suivantes (pour obtenir les composés de formules (IIc) tels que définis ci-dessus) : 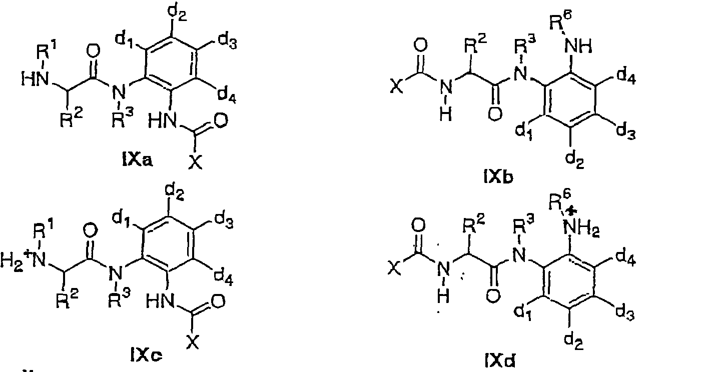 dans lesquelles
   X est tel que défini ci-dessus,
   les groupes R¹, R², R³ et R⁶ ont les significations mentionnées ci-dessus à propos des groupes R¹ à R⁵,
   les groupes d¹, d², d³ et d⁴ ont les significations mentionnées ci-dessus,
- soit à l'une des formules (Xa), (Xb), (Xc) ou (Xd) suivantes (pour obtenir les composés de formules (IId) tels que définis ci-dessus) : 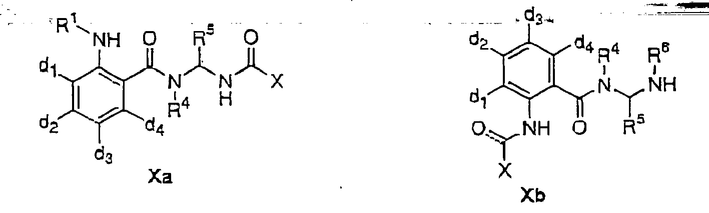 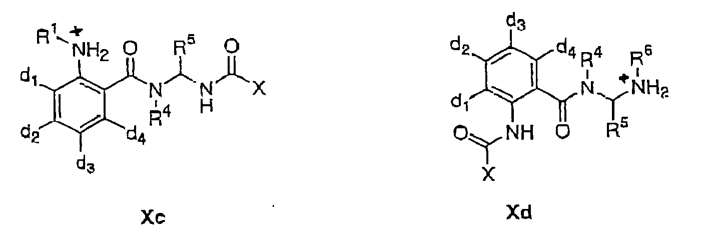 dans lesquelles
   X est tel que défini ci-dessus,
   les groupes R¹, R⁴, R⁵ et R⁶ ont les significations mentionnées ci-dessus à propos des groupes R¹ à R⁵,
   les groupes d¹, d², d³ et d⁴ ont les significations mentionnées ci-dessus,
- soit à l'une des formules (XIa), (XIb), (XIc), (XId) suivantes (pour obtenir les composés de formule (IIIa) à (IIIg) tels que définis ci-dessus) : 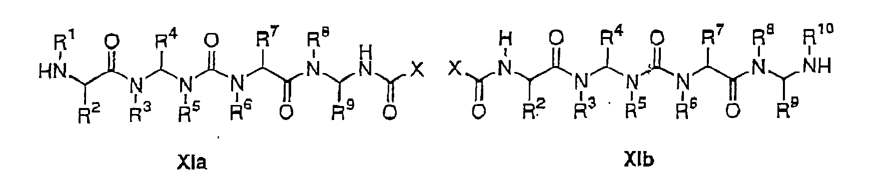 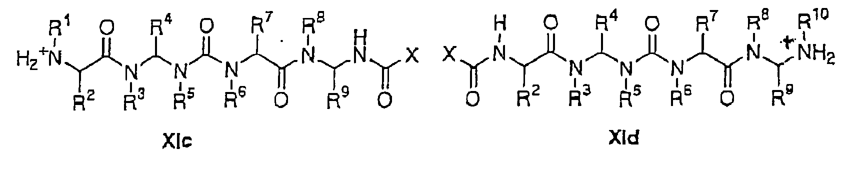 dans lesquelles
   X est tel que défini ci-dessus,
   les groupes R¹, R², R³, R⁴, R⁵, R⁶, R⁷, R⁸, R⁹ et R¹⁰ ont les significations mentionnées ci-dessus à propos des groupes R¹ à R⁵,
- soit à l'une des formules (XIIa) ou(XIIb) suivantes (pour obtenir les composés de formule (IVa) tels que définis ci-dessus) : 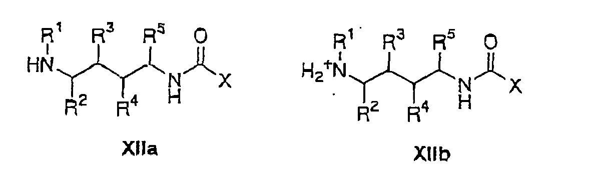 dans lesquelles
   X est tel que défini ci-dessus,
   les groupes R¹, R², R³, R⁴ et R⁵ ont les significations mentionnées ci-dessus à propos des groupes R¹ à R⁵.

Selon un mode de réalisation avantageux de la présente invention, les dérivés activés d'acide carbamique contenant une fonction amine primaire ou secondaire non protégée de formules :
(VIa), (VIb), (VIc), (VId), (VIIa), (VIIb), (VIIc), (VIId), (VIIIa), (VIIIb), (VIIIc), (VIIId), (IXa), (IXb), (IXc), (IXd), (Xa), (Xb), (Xc), (Xd), (XIa), (XIb), (XIc), (XId), (XIIa), (XIIb),
sont obtenus par libération sélective de la fonction amine protégée des dérivés activés stables d'acide carbamique correspondants dans les conditions décrites ci-dessous.

Des exemples de groupes protecteurs et de solvants de déprotection utilisés pour obtenir respectivement les composés (VIIa), (VIIb), (VIIc), (VIId), (VIIIa), (VIIIb), (VIIIc), (VIIId), (IXa), (IXb), (IXc), (IXd), (Xa), (Xb), (Xc), (Xd),(XIa), (XIb), (XIc), (XId), (XIIa), (XIIb) sont donnés ci-dessous.

La fonction amine des dérivés activés stables d'acide carbamique sera avantageusement protégée par des groupements oxycarbonyles (tels que les groupements tert-butoxycarbonyle (Boc) ou benzyloxycarbonyle (Z)) ou des groupements benzyles, ou sera masquée sous la forme de groupements nitro, cyano ou azide. Deux modes de déprotection seront utilisés avantageusement selon le procédé de l'invention.

Le groupe Boc sera déprotégé par hydrolyse acide (par exemple à l'aide d'acide trifuoroacétiquc (TFA), ou d'un mélange TFA/dichlorométhane, ou d'une solution d'acide chlorhydrique (HCl) dans un solvant organique (dioxane, éther...)), à une température d'environ 0°C à environ 40°C, afin d'obtenir les dérivés d'acide carbamique contenant une fonction amine primaire ou secondaire protonée, sous forme de trifluoroacétate ou de chlorhydrate.

L'hydrogénation sera utilisée pour le clivage des groupements 2 et benzyles, et pour la réduction des groupes nitro, cyano ou azide. L'hydrogénation pourra être effectuée avec des catalyseurs de type PtO₂, Pd/C dans des solvants comme l'éthanol, le méthanol, le diméthylformamide (DMF), l'acétate d'éthyle, le tétrahydrofuranne (THF), le chloroforme ou un mélange desdits solvants, à une température d'environ 0°C à environ 40°C, et à une pression d'environ 1 bar à environ 100 bars. En l'absence d'acide ajouté lors de l'hydrogénation (HCl ou acide acétique), la déprotection conduit exclusivement aux dérivés d'acide carbamique contenant une fonction amine primaire ou secondaire sous forme libre. L'adjonction d'un équivalent d'HCl ou d'acide acétique permet d'obtenir les dérivés d'acide carbamique contenant une fonction amine primaire ou secondaire protonée, sous forme d'acétate ou de chlorhydrate.

La cyclisation des dérivés activés d'acide carbamique contenant une fonction amine primaire ou secondaire non protégée sous forme protonée de formules :
- (VIa), (VIb), (VIc), (VId),
- (VIIa), (VIIb), (VIIc), (VIId),
- (VIIIa), (VIIIb), (VIIIc), (VIIId),
- (IXa), (IXb), (IXc), (IXd),
- (Xa), (Xb), (Xc), (Xd),
- (XIa), (XIb), (XIc), (XId),
- (XIIa), (XIIb),
pour obtenir respectivement les dérivés activés d'acide carbamique contenant de formule :
- (Ia) à (Ih),
- (IIa),
- (IIb),
- (IIc),
- (IId),
- (IIIa) à (IIIg),
- (IVa),
est effectuée respectivement dans les conditions décrites ci-dessous.

Le dérivé d'acide carbamique contenant une fonction amine primaire ou secondaire sous forme protonée, à savoir le dérivé de formule (VIc), (VId), (VIIc), (VIId), (VIIIc), (VIIId), (TXc), (IXd), (Xc), (Xd), (XIc), (XId) ou (XIIb) est
- solubilisé dans le solvant de cyclisation, notamment choisi dans le groupe constitué par l'acétonitrile (MeCN), le toluène, la pyridine, le N,N-diméthylformamide (DMF), le tétrahydrofurane (THF), le chloroforme, le dichlorométhane, la N-méthylpyrrolidone (NMP), le diméthylsulfoxide (DMSO), l'acétate d'éthyle, le méthanol, l'éthanol ou leurs mélanges, puis est :
- additionné en goutte à goutte à une solution contenant une base, notamment celle choisie dans le groupe constitué par la diisopropyléthylamine, la triéthylamine, la lutidine, la pyridine, la 2,4,6-collidine, la N-méthylmorpholine, la 2,6-di-tert-butyl-4-méthylpyridine ou leurs mélanges, et le solvant de réaction tel que défini ci-dessus,
à une température d'environ -20°C à environ 20°C, et notamment d'environ 0°C à 20°C.

La concentration du dérivé d'acide carbamique contenant une fonction amine primaire ou secondaire sous forme protonée dans le solvant de cyclisation est d'environ 10⁻⁴ M à environ 1 M, et notamment d'environ 10⁻³ M à environ 1 M.

La concentration de la base dans le solvant de cyclisation, est d'environ 10⁻⁶ M à environ 10 M, notamment d'environ 10⁻⁵ M à environ 1 M, et de préférence d'environ 10⁻⁴ M à environ 1 M.

Les conditions de cyclisation du dérivé d'acide carbamique contenant une fonction amine primaire ou secondaire sous forme libre, à savoir le dérivé de formule (VIa), (VIb), (VIIa), (VIIb), (VIIIa), (VIIIb), (IXa), (IXb), (Xa), (Xb), (XIa), (XIb) ou (XIIa), diffèrent uniquement de celles énoncées ci-dessus à propos des dérivés d'acide carbamique contenant une fonction amine primaire ou secondaire sous forme protonée, en ce qu'il n'est pas nécessaire d'opérer en présence d'une base.

Selon un autre mode de réalisation avantageux, l'invention concerne un procédé de préparation tel que défini ci-dessus, de composés urées cycliques de formule (IIIa) à (IIIg) tels que définis ci-dessus, caractérisé en ce que lesdits composés (IIIa) à (IIIg) sont obtenus à l'issue d'une réaction d'homo-oligomérisation ou d'hétéro-oligomérisation, à partir d'au moins un dérivé activé d'acide carbamique contenant une fonction amine primaire ou secondaire, répondant à l'une au moins des formules (VIa), (VIb), (VIc) ou (VId) telles que définies ci-dessus.

La réaction d'homo-oligomérisation ou d'hétéro-oligomérisation des dérivés activés d'acide carbamique contenant une fonction aminé primaire ou secondaire de formule (VIa), (VIb), (VIc) ou (VId), ainsi que la cyclisation des homo ou hétéro-oligomères ainsi obtenus en composés urées cycliques de formule (IIIa) à (IIIg), est favorisée pour des dérivés d'acide carbamique de formule (VIa), (VIb), (VIc) ou (VId) dans lesquels la liaison -CO-NR³ adopte majoritairement une conformation trans.

La réaction d'homo-oligomérisation ou d'hétéro-oligomérisation est avantageusement effectuée pour des faibles concentrations des dérivés activés d'acide carbamique de formule (VIa), (VIb), (VIc) ou (VId) dans le solvant de cyclisation, à savoir des concentrations d'environ 10⁻³ M à environ 10⁻⁵ M, à des températures d'environ 0°C à environ 20°C.

Selon un mode de réalisation avantageux, le procédé de préparation de l'invéntion cst caractérisé en ce qu'il comporte :
◆ une étape de cyclisation des dérivés activés d'acide carbamique contenant une fonction amine primaire ou secondaire non protégée,
   conduisant à des composés urées cycliques comportant dans leur cycle une fonction urée de formule -NH-CO-N< ou -NH-CO-NH-,
◆ une étape d'alkylation de l'hydrogène du ou des groupes -NH- compris dans la fonction urée du composé urée cyclique obtenu à l'issue de l'étape de cyclisation ci-dessus.

L'étape d'alkylation de l'hydrogène du ou des groupes -NH- compris dans la fonction urée du composé urée cyclique obtenu à l'issue de l'étape de cyclisation consiste à faire réagir un agent alkylant sur la ou les fonctions NH de l'urée cyclique en présence d'une base adaptée.

A titre d'exemple d'un agent alkylant, on pourra notamment citer un dérivé halogéné, le groupement halogène étant généralement un chlore, un brome, ou un iode.

A titre d'exemple d'une base, on pourra notamment citer celle choisie dans le groupe constitué par :
- un hydrure de métal, tel qu'un hydrure de sodium (NaH),
- un alcoolat de métal tel que le méthanolate de sodium ou le t-butanolate de potassium,
- le bis(triméthylsilyl)amide de sodium, de lithium ou de potassium,
- le fluorure de potassium sur alumine (KF/Al₂O₃),
dans des solvants non protiques tels que le tétrahydrofurane (THF), le N,N-diméthylformamide (DMF), la N-méthylpyrrolidone, le diméthoxy éthane (DME), ou dans des conditions de transfert de phase avec le carbonate de potassium (K₂CO₃), le carbonate de sodium (Na₂CO₃) ou la potasse (KOH).

Selon un mode de réalisation avantageux, le procédé de préparation des composés urées cycliques de formule (Ia), (Ib), (Ic), (Id), (Ie), (If), (Ig) et (Ih) tels que décrit ci-dessus est plus particulièrement caractérisé en ce qu'il comporte :
◆ une étape de cyclisation :
   - des dérivés activés d'acide carbamique contenant une fonction amine primaire ou secondaire non protégée de formule (VIa) ou (VIc) ou,
   - des dérivés activés d'acide carbamique contenant une fonction amine primaire ou secondaire non protégée de formule (VIb) ou (VId),
   conduisant respectivement à :
   - des composés urées cycliques comportant dans leur cycle une fonction urée de formule -NH-CO-NR¹- ou,
   - des composés urées cycliques comportant dans leur cycle une fonction urée de formule -R⁵N-CO-NH-,
◆ une étape d'alkylation respectivement :
   - de l'hydrogène de la fonction urée de formule -NH-CO-NR¹- obtenue à l'issue de l'étape de cyclisation à l'aide d'un agent alkylant comportant le groupe R⁵ ou,
   - de l'hydrogène de la fonction urée de formule -R⁵N-CO-NH- obtenue à l'issue de l'étape de cyclisation à l'aide d'un agent alkylant comportant le groupe R¹,
afin d'obtenir les composés urées cycliques de formule (Ia), (Ib), (Ic), (Id), (Ie), (If), (Ig) et (Ih) comportant dans leur cycle la fonction urée -(R⁵N-CO-NR¹)-, les groupes R¹ et R⁵ étant tels que décrits ci-dessus.

Selon un mode de réalisation avantageux, le procédé de préparation des composés urées cycliques de formule (IIa) est plus particulièrement caractérisé en ce qu'il comporte :
◆ une étape de cyclisation :
   - des dérivés activés d'acide carbamique contenant une fonction amine primaire ou secondaire non protégée de formule (VIIa) ou (VIIc) ou,
   - des dérivés activés d'acide carbamique contenant une fonction amine primaire ou secondaire non protégée de formule (VIIb) ou (VIId),
   conduisant respectivement à :
   - des composés urées cycliques comportant dans leur cycle une fonction urée de formule -NH-CO-NR¹- ou,
   - des composés urées cycliques comportant dans leur cycle une fonction urée de formule -R⁶N-CO-NH-,
◆ une étape d'alkylation respectivement :
   - de l'hydrogène de la fonction urée de formule -NH-CO-NR¹- obtenue à l'issue de l'étape de cyclisation à l'aide d'un agent alkylant comportant le groupe R⁶ ou,
   - de l'hydrogène de la fonction urée de formule -R⁶N-CO-NH- obtenue à l'issue de l'étape de cyclisation à l'aide d'un agent alkylant comportant le groupe R¹,
afin d'obtenir les composés urées cycliques de formule (IIa) comportant dans leur cycle la fonction urée -(R⁶N-CO-NR¹)-, les groupes R¹ et R⁶ étant tels que décrits ci-dessus.

Selon un mode de réalisation avantageux, le procédé de préparation des composés urées cycliques de formule (IIb) est plus particulièrement caractérisé en ce qu'il comporte :
◆ une étape de cyclisation :
   - des dérivés activés d'acide carbamique contenant une fonction amine primaire ou secondaire non protégée de formule (VIIIa) ou (VIIIc) ou,
   - des dérivés activés d'acide carbamique contenant une fonction amine primaire ou secondaire non protégée de formule (VIIIb) ou (VIIId),
   conduisant respectivement à :
   - des composés urées cycliques comportant dans leur cycle une fonction urée de formule -NH-CO-NR¹- ou,
   - des composés urées cycliques comportant dans leur cycle une fonction urée de formule -R⁶N-CO-NH-,
◆ une étape d'alkylation respectivement:
   - de l'hydrogène de la fonction urée de formule -NH-CO-NR¹- obtenue à l'issue de l'étape de cyclisation à l'aide d'un agent alkylant comportant le groupe R⁶ ou,
   - de l'hydrogène de la fonction urée de formule -R⁶N-CO-NH- obtenue à l'issue de l'étape de cyclisation à l'aide d'un agent alkylant comportant le groupe R¹,
afin d'obtenir les composés urées cycliques de formule (IIb) comportant dans leur cycle la fonction urée -(R⁶N-CO-NR¹)-.

Selon un mode de réalisation avantageux, le procédé de préparation des composés urées cycliques de formule (IIc) est plus particulièrement caractérisé en ce qu'il comporte.
◆ une étape de cyclisation :
   - des dérivés activés d'acide carbamique contenant une fonction amine primaire ou secondaire non protégée de formule (IXa) ou (XXc) ou,
   - des dérivés activés d'acide carbamique contenant une fonction aminé primaire ou secondaire non protégée de formule (IXb) ou (IXd),
   conduisant respectivement à :
   - des composés urées cycliques comportant dans leur cycle une fonction urée de formule -NH-CO-NR¹- ou,
   - des composés urées cycliques comportant dans leur cycle une fonction urée de formule -R⁶N-CO-NH-,
◆ une étape d'alkylation respectivement :
   - de l'hydrogène de la fonction urée de formule -NH-CO-NR¹- obtenue à l'issue de l'étape de cyclisation à l'aide d'un agent alkylant comportant le groupe R⁶ ou,
   - de l'hydrogène de la fonction urée de formule -R⁶-CO-NH- obtenue à l'issue de l'étape de cyclisation à l'aide d'un agent alkylant comportant le groupe R¹,
afin d'obtenir les composés urées cycliques de formule (IIc) comportant dans leur cycle la fonction urée -(R⁶N-CO-NR¹)-.

Selon un mode de réalisation avantageux, le procédé de préparation des composés urées cycliques de formule (IId) est plus particulièrement caractérisé en ce qu'il comporte :
◆ une étape de cyclisation :
   - des dérivés activés d'acide carbamique contenant une fonction amine primaire ou secondaire non protégée de formule (Xa) ou (Xc) ou,
   - des dérivés activés d'acide carbamique contenant une fonction amine primaire ou secondaire non protégée de formule (Xb) ou (Xd),
   conduisant respectivement à :
   - des composés urées cycliques comportant dans leur cycle une fonction urée de formule -NH-CO-NR¹- ou,
   - des composés urées cycliques comportant dans leur cycle une fonction urée de formule -R⁶N-CO-NH-,
◆ une étape d'alkylation respectivement :
   - de l'hydrogène de la fonction urée de formule -NH-CO-NR¹- obtenue à l'issue de l'étape de cyclisation à l'aide d'un agent alkylant comportant le groupe R⁶ ou,
   - de l'hydrogène de la fonction urée de formule -R⁶N-CO-NH- obtenue à l'issue de l'étape de cyclisation à l'aide d'un agent alkylant comportant le groupe R¹,
afin d'obtenir les composés urées cycliques de formule (IId) comportant dans leur cycle la fonction urée -(R⁶N-CO-NR¹)-.

Selon un mode de réalisation avantageux, le procédé de préparation des composés urées cycliques de formule (IIIa), (IIIb), (IIIc), (IIId), (IIIe), (IIIf) et (IIIg) est plus particulièrement caractérisé en ce qu'il comporte :
◆ une étape de cyclisation :
   - des dérivés activés d'acide carbamique contenant une fonction amine primaire ou secondaire non protégée de formule (XIa) ou (XIc) ou,
   - des dérivés activés d'acide carbamique contenant une fonction amine primaire ou secondaire non protégée de formule (XIb) ou (XId),
   conduisant respectivement à :
   - des composés urées cycliques comportant dans leur cycle une fonction urée de formule -NH-CO-NR¹- ou,
   - des composés urées cycliques comportant dans leur cycle une foriction urée de formule -R¹⁰N-CO-NH-,
◆ une étape d'alkylation respectivement :
   - de l'hydrogène de la fonction urée de formule -NH-CO-NR¹- obtenue à l'issue de l'étape de cyclisation à l'aide d'un agent alkylant comportant le groupe R¹⁰ ou,
   - de l'hydrogène de la fonction urée de formule -R¹⁰N-CO-NH- obtenue à l'issue de l'étape de cyclisation à l'aide d'un agent alkylant comportant le groupe R¹,
afin d'obtenir les composés urées cycliques de formule (IIIa), (IIIb), (IIIc), (IIId), (IIIe), (IIIf) et (IIIg) comportant dans leur cycle la fonction urée -(R¹⁰N-CO-NR¹)-, les groupes R¹ et R¹⁰ étant tels que décrits ci-dessus.

Selon un mode de réalisation avantageux, le procédé de préparation des composés urées cycliques de formule (IVa) est plus particulièrement caractérisé en ce qu'il comporte :
◆ une étape de cyclisation des dérivés activés d'acide carbamique contenant une fonction amine primaire ou secondaire non protégée de formule (XIIa) ou (XIIb),
   conduisant à des composés urées cycliques comportant dans leur cycle une fonction urée de formule -NH-CO-NR¹-,
◆ une étape d'alkylation de l'hydrogène de la fonction urée de formule -NH-CO-NR¹- obtenue à l'issue de l'étape de cyclisation à l'aide d'un agent alkylant comportant le groupe R⁶,
afin d'obtenir les composés urées cycliques de formule (IVa) comportant dans leur cycle la fonction urée -(R⁶N-CO-NR¹)-.

L'étape d'alkylation du procédé de préparation des composés urées cycliques tels que décrits ci-dessus, pourra notamment être effectuée dans les conditions particulières décrites ci-après. Une solution d'un composé urée cyclique (10 mmol) dans le THF (10 ml) est ajoutée goutte à goutte à une suspension de NaH (1-1.2 équivalents si un NH à alkyler, 2-2.4 équivalents si deux NH à alkyler) dans le THF (sous Argon et à 0°C). Le milieu réactionnel est agité à 0°C pendant 60 minutes puis l'agent alkylant (1-1.5 équivalents si un NH à alkyler, 2-3 équivalents si deux NH à alkyler) dissous dans le THFest ajouté à 0°C. La réaction est laissée pendant 12h puis le milieu réactionnel est dilué avec de l'acétate d'éthyle et avec une solution de NH₄Cl saturée. La phase organique est lavée avec une solution saturée de KHSO₄ 1N, H₂O, une solution de NaHCO₃ saturé, HzO. La phase organique est séchée sur MgSO4 et le solvant est concentré par évaporation rotative.

L'invention concerne également un procédé de préparation tel que défini ci-dessus de composés urées cycliques comportant un cycle à 6 atomes et comprenant une fonction urée, d.e formule (Va) : 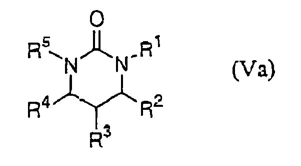 dans laquelle R¹, R², R³, R⁴ et R⁵ peuvent représenter chacun et indépendamment les uns des autres :
a) - un hydrogène,
b) - un halogène,
c) - la chaîne latérale protégée ou non d'un acide aminé choisi parmi les acides aminés naturels ou non naturels,
d) - un groupe alkyle (C1-C20), linéaire ou ramifié, non substitué ou substitué par un ou plusieurs substituants parmi lesquels : -COORₐ, -CONHRₐ, -ORₐ, -NHRₐ, -NH(CO)Rₐ, -NHCOORₐ, un groupement aryle ou hétéroaryle, dont la structure cyclique contient de 5 à 20 atomes de carbone, un atome d'halogène, un groupement R'''CO-, le groupe R''' comprenant de 1 à 10 atomes de carbone, un groupe nitrile, guanidino ou nitro,
e) - un groupement aryle dont la structure du cycle contient de 5 à 20 atomes de carbone, substitué ou non par les substituants mentionnés ci-dessus, ainsi que par les groupements cyano ou amidine,
f) - un groupement alkényle ou alkynyle (C1-C6)
g) - un groupement sulfonyle (R_{c}SO2)
h) - un groupement acyle (R_{c}CO)
i) - un groupement OR_{b}
j) - un groupement NH₂
k) -COOR_{b}
1) -CONHR_{b}
m) -CH₂CONH₂
Rₐ et R_{b} représentant indépendamment l'un de l'autre un hydrogène, un groupe allyle, benzyle, 1-butyle, fluorénylméthyle, benzyloxyméthyle, tert-butyldiméthylsilyle, 2-éthoxyéthyle, méthoxyméthyle, 2-méthoxyéthoxyméthyle, tétrahydropyran-2-yle, triméthylsilyle, triéthylsilyle, 2-(triméthylsilyl)éthyle, trityle, 2,2,2-trichloroéthyle, tosyle, ortho-(ou para)-nitrophénylsulfonylc, alkyle ayant de 1 à 20 atomes de carbone, ou un groupe aryle dont la structure du cycle contient de 5 à 20 atomes de carbone,
R_{c} représentant un groupement alkyle ayant de 1 à 20 atomes de carbone, ou un groupe aryle dont la structure du cycle contient de 5 à 20 atomes de carbone, ou un groupe hétéroaryle, arylalkyle ou hétéroarylalkyle,
les groupes R¹, R², R³ et R⁴ pouvant former les cyclisations intramoléculaires suivantes :
1/ cyclisation entre R¹ et R² et/ou,
2/ cyclisation entre R³ et R⁴,
lesdits composés urées cycliques pouvant être, lorsqu'un ou plusieurs carbones asymétriques sont présents dans la formule (Va), de façon indépendante, soit de configuration R (rectus) soit de configuration S (sinister),
à partir d'un dérivé activé d'acide carbamique contenant une fonction amine primaire ou secondaire répondant à l'une des formules (XIIIa) ou (XIIIb) suivantes : 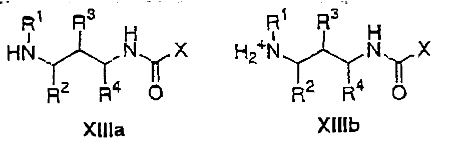 dans lesquelles
le groupe X représente un groupe conférant au dérivé une structure de dérivé activé d'acide carbamique, lequel groupe X étant issu d'un composé choisi notamment parmi les phénols, éventuellement substitués par au moins un groupe nitro ou au moins un halogène, ou les dérivés d'hydroxylamine, ou les dérivés d'alcools benzyliques greffés sur support solide et plus particulièrement choisi parmi les composés suivants : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzotriazine (HODhbt), 7-aza-1-hydroxy-benzotriazole (HOAt), 4-aza-1-hydroxybenzotriazole (4-HOAt), imidazole, tétrazole, résine WANG, et
les groupes R¹, R², R³ et R⁴ sont tels que définis ci-dessus.

L'invention a également pour objet un procédé de préparation tel que défini ci-dessus, de composés urées cycliques comportant un cycle ayant au moins 8 atomes, lequel cycle comprenant au moins deux fonctions urées espacées l'une de l'autre par au moins un atome de carbone et notamment par 1 à 4 atomes de carbone.

A cet égard, l'invention a plus particulièrement pour objet un procédé de préparation tel que défini ci-dessus, de composés urées cycliques comportant un cycle ayant au moins 8 atomes et comprenant au moins deux fonctions urées espacées l'une de l'autre par 2 atomes de carbones, répondant aux formules (XIV), (XV) et (XVIa) : 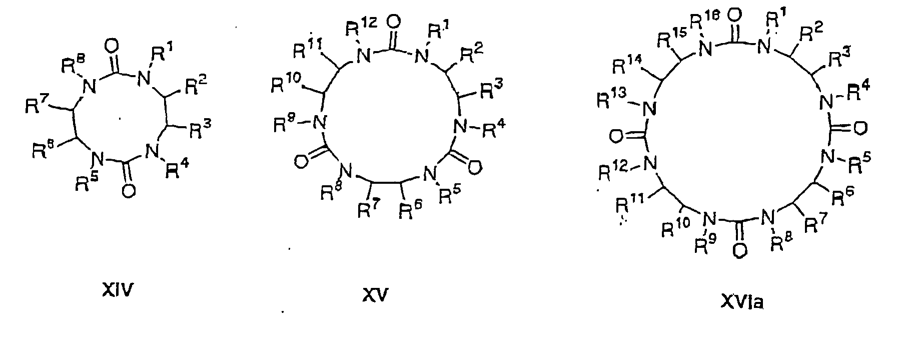 dans lesquelles les groupes R¹, R², R³, R⁴, R⁵, R⁶, R⁷, R⁸, R⁹, R¹⁰; R¹¹, R¹², R¹³, R¹⁴, R¹⁵ et R¹⁶ ont les significations mentionnées ci-dessus à propos des groupes R¹ à R⁵,
les groupes R¹, R², R⁵, R⁶, k⁹, R¹⁰, R¹³ et R¹⁴ pouvant également former les cyclisations intramoléculaires suivantes :
1/ cyclisation entre R¹ et R² et/ou,
2/ cyclisation entre R⁵ et R⁶ et/ou,
3/ cyclisation entre R⁹ et R¹⁰ et/ou,
4/ cyclisation entre R¹³ et R¹⁴,
à partir respectivement des dérivés activés d'acide carbamique contenant une fonction amine primaire ou secondaire et répondant aux formules (XXIII), (XXIV) et (XXV) suivantes : 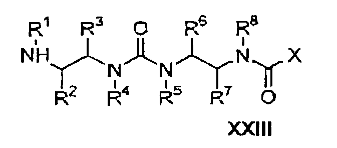 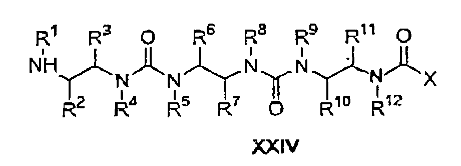 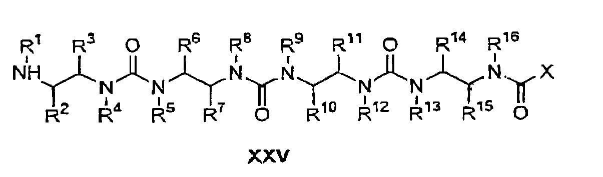 dans lesquelles
les groupes R¹, R², R³, R⁴, R⁵, R⁶, R⁷, R⁸, R⁹, R¹⁰, R¹¹, R¹², R¹³, R¹⁴, R¹⁵ et R¹⁶ sont tels que définis ci-dessus,
le groupe X est tel que défini ci-dessus,
lesdits dérivés d'acide carbamique pouvant également être sous forme protonée.

L'intention a encore pour objet un procédé de préparation tel que défini ci-dessus, de composés urées cycliques comportant au moins quatre fonctions urée, de formules (XVIb), (XVIc), (XVId) et (XVIe) : 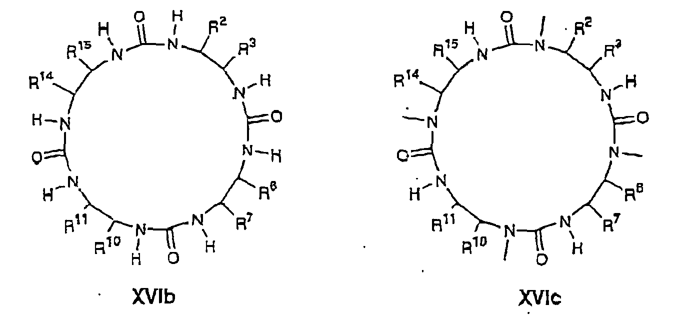 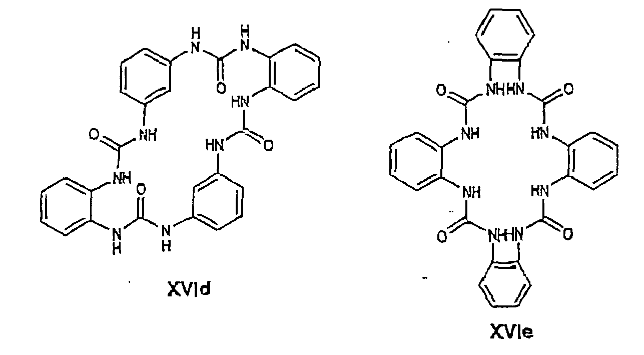 dans lesquelles
les groupes R², R³, R⁶, R⁷, R⁸, R¹⁰, R¹¹, R¹⁴ et R¹⁵ ont les significations mentionnées ci-dessus à propos des groupes R¹ à R⁵.

L'invention a également pour objet un procédé de préparation tel que défini ci-dessus, de composés de formule (XVIb), dans laquelle:
- les substituants R², R⁵, R¹⁰ et R¹⁴ sont choisis parmi :
   * un atome d'hydrogène,
   * une chaîne alkyle C1-C6, linéaire ou ramifiée, substituée ou non par :
      i) une fonction amine protégée ou non,
      ii) une fonction acide protégée ou non,
      iii) une fonction alcool protégée ou non,
      iv) un groupement aryle ou hétéroaryle,
   * la chaîne latérale protégée ou non d'un acide aminé choisi parmi les acides aminés naturels ou non naturels
- les substituants R³, R⁷, R¹¹ et R¹⁵ représentent un atome d'hydrogène,
à partir de dérivés activés d'acide carbamique de formule (XXV), telle que mentionnée ci-dessus, dans laquelle :
i) - les substituants R², R⁶, R¹⁰ et R¹⁴ ont la même définition que ci-dessus et les substituants R¹, R³, R⁴, R⁵, R⁷, R⁸, R⁹, R¹¹, R¹², R¹⁵ et R¹⁶ représentent un atome d'hydrogène, ou
   - les substituants R³, R⁷, R¹¹ et R¹⁵ ont les significations mentionnées précédemment pour R², R⁶, R¹⁰ et R¹⁴ et les substituants R¹, R², R⁴, R⁵, R⁶, R⁸, R⁹, R¹⁰, R¹², R¹⁴ et R¹⁶ représentent un atome d'hydrogène, et
ii) le groupement R¹³ est un groupe arylalkyle ou hétéroarylalkyle, qui peut être relié à un support solide,
et permettant la formation, avant l'étape finale de déprotection, d'un intermédiaire de synthèse ayant la formule (XVIa), telle que mentionnée ci-dessus, dans laquelle les différents substituants R¹ à R¹⁶ ont les mêmes significations que mentionnées ci-dessus pour le composé de formule (XXV).

L'invention a également pour objet un procédé de préparation tel que défini ci-dessus, de composés de formule (XV), dans laquelle :
- les substituants R², R⁶ et R¹⁰ sont choisis parmi :
   * un atome d'hydrogène
   * une chaîne alkyle C1-C7, linéaire ou ramifiée, substituée ou non par :
      i) une fonction aminé protégée ou non,
      ii) une fonction acide protégée ou non,
      iii) une fonction alcool protégée ou non,
      iv) un groupement aryle ou hétéroaryle,
   * la chaîne latérale protégée ou non d'un acide aminé choisi parmi les acides aminés naturels ou non naturels,
- les substituants R¹, R³, R⁴, R⁵, R⁷, R⁸, R⁹, R¹¹ et R¹² représentent un atome d'hydrogène,
à partir de dérivés activés d'acide carbamique de formule (XXIV), telle que mentionnée ci-dessus, dans laquelle :
i) - les substituants R², R⁶ et R¹⁰ ont les significations mentionnées ci-dessus et les substituants R¹, R³, R⁴, R⁵, R⁷, R⁸, R¹¹ et R¹² représentent un atome d'hydrogène, ou
   - les substituants R³, R⁷ et R¹¹ ont les significations mentionnées précédemment pour R², R⁶ et R¹⁰, et les substituants R¹, R², R⁴, R⁵, R⁶, R⁸, R¹⁰ et R¹² représentent un atome d'hydrogène,
ii) le groupement R⁹ est un groupe arylalkyle ou hétéroarylalkyle, qui peut être relié à un support solide,
et permettant la formation, avant l'étape finale de déprotection, d'un intermédiaire de synthèse ayant la formule (XV), telle que mentionnée ci-dessus, dans laquelle les différents substituants R¹ à R¹² ont les mêmes significations que mentionnées ci-dessus pour le composé de formule (XXIV).

L'invention concerne également des composés de formules (XV) ou (XVIa) telles que mentionnées ci-dessus, dans lesquelles les substituants R¹, R², R³, R⁴, R⁵, R⁶, R⁷, R⁸, R⁹, R¹⁰, R¹¹, R¹², R¹³, R¹⁴, R¹⁵ et R¹⁶ représentent :
- soit un atome d'hydrogène,
- soit la chaîne latérale protégée ou non d'un acide aminé choisi parmi les acides aminés naturels ou non naturels,
et notamment dans lesquels :
* R¹, R³, R⁴, R⁵, R⁷, R⁸, R⁹, R¹¹, R¹², R¹³, R¹⁵ et R¹⁶ représentent un atome d'hydrogène, et
* R², R⁶, R¹⁰ et R¹⁴ représentent un groupe choisi parmi les groupes méthyle, isopropyle, isobutyle, sec-butyle, benzyle, acétate d'alkyle et hydroxybenzyle (ortho, méta ou para),
sous réserve que les composés de formule (XV) ou (XVIa) soient différents des composés suivants de formules (VIII bis/1), (VIII bis/2), (VIII bis/3) et (VIII bis/4)

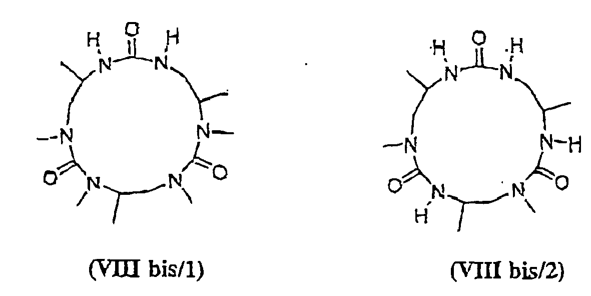

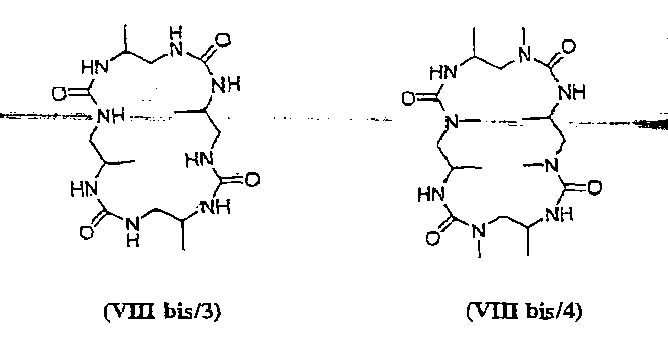

L'invention concerne également des composés tels que définis ci-dessus, répondant aux formules suivantes : 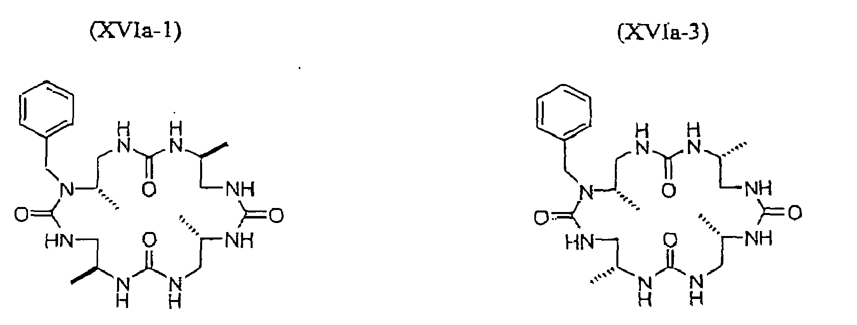 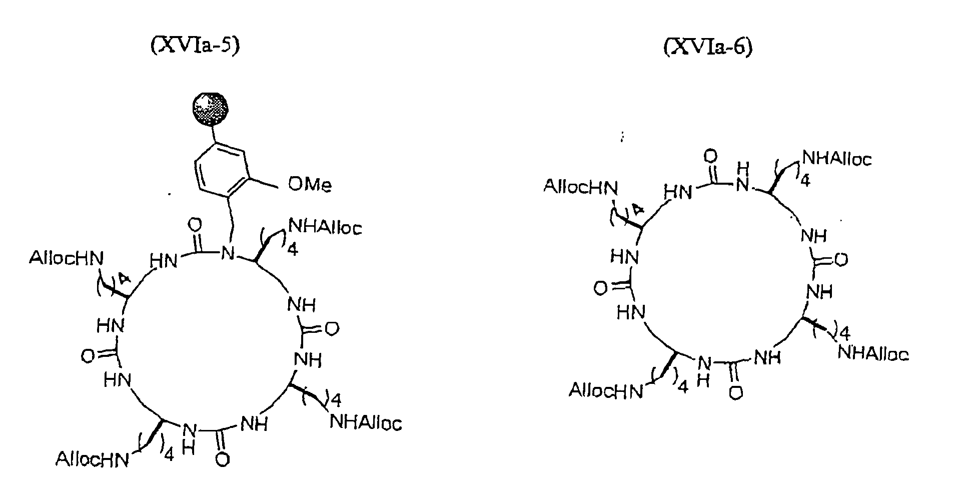 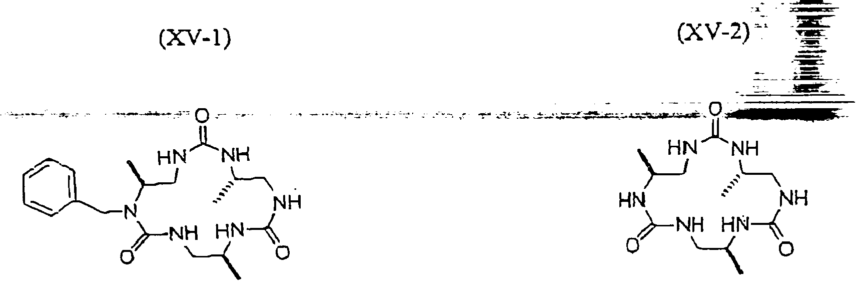 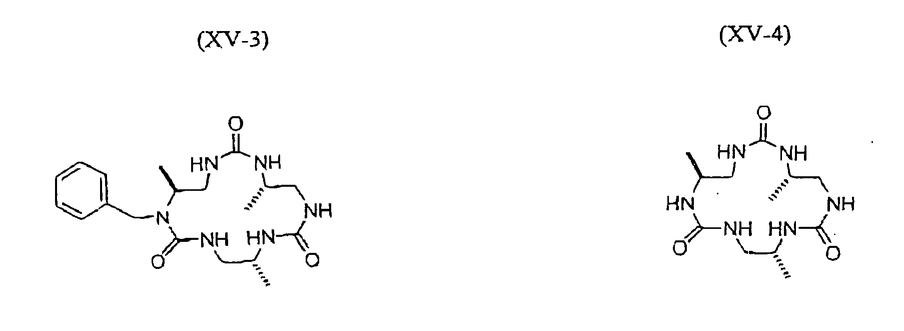 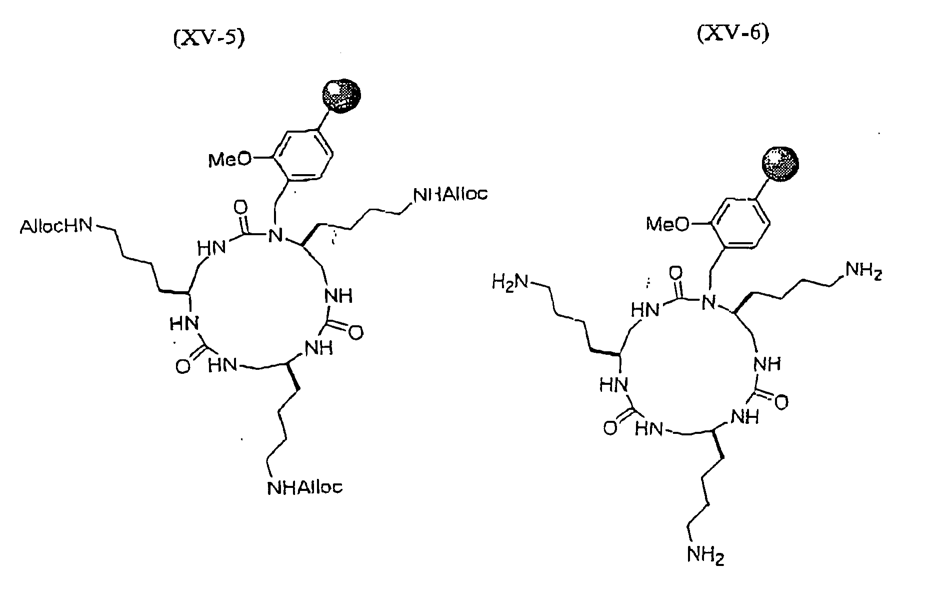 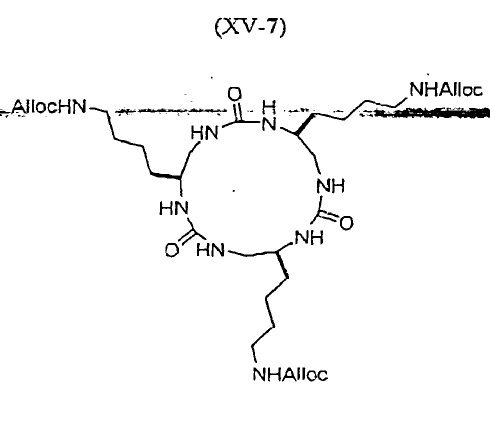 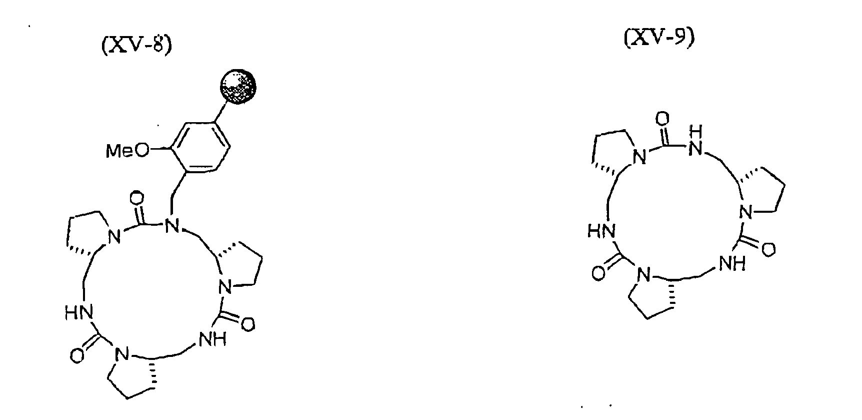 dans lesquelles Alloc représente un groupe allyloxycarbonyle et

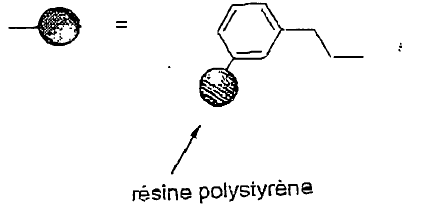

Selon un mode de réalisation avantageux, le procédé de préparation de la présente invention permet notamment d'obtenir de nouveaux composés urées cycliques qui auraient été difficilement obtenus par les procédés de préparation de l'art antérieur qui nécessitent en général la préparation de diamines. Le procédé de préparation de la présente invention permet la cyclisation de dérivés d'acide carbamique obtenus à partir de dérivés d'acides aminés N-protégés (acides aminés α, β, γ et δ), et permet donc d'obtenir facilement et en très peu d'étapes une grande diversité moléculaire sur les chaînes latérales utilisées. Le procédé de l'invention s'applique également à la cyclisation de dérivés d'acide carbamique obtenus en seulement trois étapes, à savoir :
a) une étape de transformation du groupe -COOH du dérivé d'acide aminé N-protégé (acides aminés α, β, γ et δ) en groupe -CON₃ pour obtenir un acyl azide,
b) une étape de transformation du groupe -CON₃ de l'acyl azide en groupe -NCO pour obtenir un isocyanate,
c) une étape de traitement de l'isocyanate pour obtenir le susdit dérivé stable d'acide carbamique,
et ceci à partir de molécules très simples, comme les dipeptides N-protégés, pour donner des molécules urées cycliques extrêmement fonctionnalisées et dissymétriques.

Rappelons que le termé "dérivé d'acide aminé" doit s'interpréter au sens large, tel que compris par l'homme du métier, et désigne notamment un dérivé de peptide, de polypeptide, de protéine, de pseudopeptide ou d'oligourée.

### Description des figures

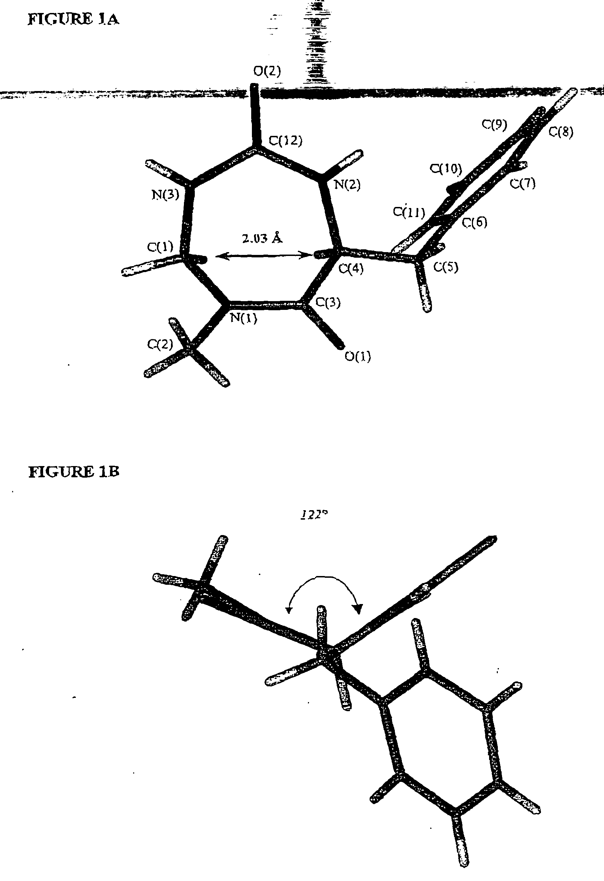

Les Figures 1A et 1B représentent la structure bidimensionnelle du composé de formule (Ij). La Figure 1A correspond à la vue au-dessus du cycle urée.
La Figure 1B correspond à la vue le long de l'axe formé par les carbones C^{α}, notés C(1) et C(4).

Pour plus de clarté, les atomes ont été numérotés. Les tableaux suivants indiquent certaines distances interatomiques (en Å) et certaines valeurs angulaires.

**Longueurs de certaines liaisons dans le composé de formule (Ij)**

| Liaison | Longueur (Å) |
|---|---|
| N(1) - C(3) | 1,32 |
| N(2) - C(12) | 1,32 |
| C(12) - N(3) | 1,38 |

**Valeurs de certains angles dans le composé de formule (Ij)**

| Angle | Valeur (°) |
|---|---|
| C(3)-N(1)-C(1) | 120 |
| N(1)-C(3)-C(4) | 119 |
| C(12)-N(2)-C(4) | 132 |
| N(2) - C(12)-N(3) | 119 |
| C(12)-N(3)-C(1) | 126 |

**Valeurs de certains angles de torsion dans le composé de formule (Ij)**

| Angle | Valeur (°) |
|---|---|
| N(3) - C(12) - N(2) - C(4) | 0,47 |
| C(1) - N(3) - C(12) - N(2) | -3,43 |
| C(4) - C(3) - N(1) - C(1) | -1,07 |

Les exemples ci-après illustrent l'invention. Ils ne la limitent en aucune façon.

### Exemple 1

### Réactions intermoléculaires et intramoléculaires pouvant avoir lieu lors de la préparation des composés urées cycliques.

Le processus de macrocyclisation à partir de précurseurs acycliques bifonctionnels (dérivés activés d'acide carbamique contenant une fonction amine primaire ou secondaire) homo-oligomériques et/ou hétéro-oligomériques, peut conduire à l'obtention de composés urées cycliques homo-oligomériques et/ou hétéro-oligomériques, dont la distribution en taille dépend de la dilution du milieu réactionnel et de la molarité effective des différents précurseurs linéaires. C'est la compétition qui existe entre les processus de réactions intermoléculaires et intramoléculaires qui conduit à l'obtention d'un mélange réactionnel pouvant être plus ou moins complexe.

### 1) Utilisation d'un dérivé activé stable d'acide carbamique

Lorsque le dérivé activé d'acide carbamique contenant une fonction amine primaire ou secondaire sous forme protonée (précurseur) est mis à réagir en présence d'une hase, il est possible d'obtenir des urées cycliques homo-oligomériques de tailles variables, et dans des proportions variables.

Le schéma 1 ci-dessous représente les composés urées cycliques homo-oligomériques obtenus à l'issue de réactions intermoléculaires et intramoléculaires.

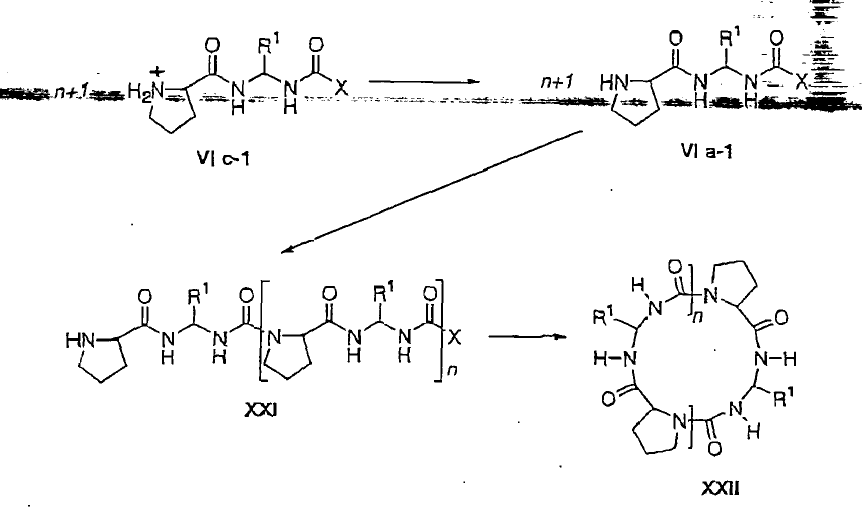

Dans ce schéma, les fonctions amines secondaires des dérivés activés d'acide carbamique (précurseurs acycliques bifonctionnels) sont représentées sous forme protonée **(VIc-1)** et sous forme libre **(VIa-1).**

Lorsque le composé de départ est le précurseur acyclique bifonctionnel **(VIa-1)** (dérivé activé d'acide carbamique contenant une fonction amine secondaire sous forme libre) obtenu à l'issue de l'étape de libération, la réaction intermoléculaire d'homo-oligoménsation peut avoir lieu directement après ladite étape de libération, sans l'ajout d'une base.

Lorsque le composé de départ est le précurseur acyclique bifonctionnel **(VIe-1)** (dérivé activé d'acide carbamique contenant une fonction amine secondaire sous forme protonée) la réaction intermoléculaire d'homo-oligomérisation a lieu en présence d'une base, afin de neutraliser la fonction amine secondaire protonée en fonction amine secondaire sous forme libre **(VIa-1).** On obtient, à l'issue de la réaction intermoléculaire d'homo-oligomérisation, un précurseur acyclique bifonctionnel homo-oligomérique contenant une fonction amine secondaire sous forme libre **(XXI),** qui subit une cyclisation intramoléculaire pour former une urée cyclique homo-oligomérique **(XXII).**

Ainsi, dans le cas où le précurseur est la molécule **(VIc-1)** ou **(VIa-1),** ou l'homo-oligomère **(XXI),** la spectrométrie de masse du milieu réactionnel permet de détecter des urées cycliques **(XXII)** de tailles variables, allant du monomère à l'octamère et représentées à l'aide du nombre entier n (n = entier allant de 0 à 7). Lorsque n est égal à 0, il n'y a pas formation d'homo-oligomère, et l'étape b) de cyclisation intramoléculaire a lieu immédiatement après la formation du composé (**VIa-1**).

En général pour cette famille de précurseurs, parmi les urées cycliques présentes dans le milieu réactionnel, le dimèze cyclique (n = 1) est le produit majoritaire.

Il est donc possible à partir d'un précurseur unique (dérivé activé stable d'acide carbamique contenant une fonction amine primaire ou secondaire sous forme libre ou sous forme protonée) d'obtenir un mélange d'urées cycliques bomo-oligomériques présentant des cycles de différentes tailles. Les urées cycliques ainsi obtenues peuvent être caractérisées par spectrométrie de masse et purifiées par chromatographie.

### 2) Utilisation de deux dérivés activés stables d'acide carbamique

Il est également possible, en partant d'un mélange de plusieurs précurseurs de la même famille, d'obtenir une distribution statistique, pour les différentes tailles de cycle, d'urées cycliques hétéro-oligomériques et homo-oligomériques.

Le schéma 2 ci-dessous représente des composés urées cycliques homodimériques **(IIIf1)** et **(IIIf3)** et hétérodimériques **(IIIf2)** obtenus à l'issue de réactions intermoléculaires et intramoléculaires.

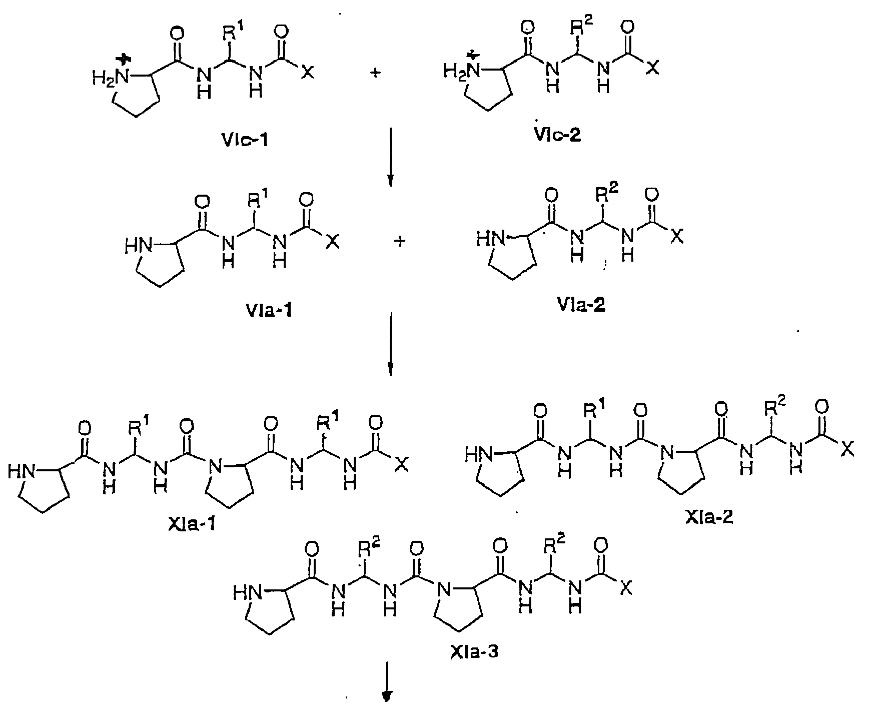

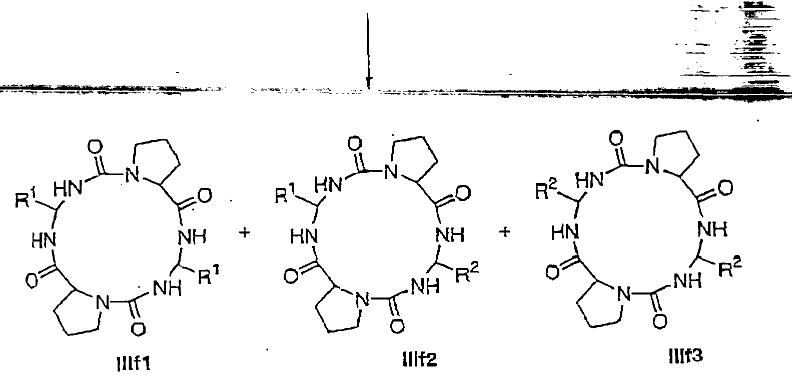

Les fonctions amines secondaires des précurseurs acycliques bifonctionnels sont représentées sous forme protonée (**VIc-1**) et (**VIc-2**) et sous forme libre (**VIa-1**) et (**VIa-2**).

Les deux précurseurs acycliques bifonctionnels (**VIc-1**) et (**VIc-2**) (dérivés activés d'acide carbamique contenant une fonction amine secondaire sous forme protonée) subissent chacun, en présence d'une base, une homo-dimérisation pour former respectivement deux précurseurs acycliques bifonctionnels homodimériques (**XIa-1**) et (**XIa-3**) contenant une fonction amine secondaire sous forme libre, ainsi qu'une hétéro-dimérisation pour former un précurseur bifonctionnel dimérique (**XIa-2**) contenant une fonction amine secondaire sous forme libre.

Lesdits dérivé homo-dimériques (**XIa-1**), (**XIa-3**) et hétérodimérique (**XIa-2**) ainsi obtenus subissent une cyclisation intramoléculaire pour former respectivement des urées cycliques homodimériques (**IIIf1**) et (**IIIf3**) et une urée cyclique hétérodimérique **(IIIf2)**.

Ainsi, si la réaction est conduite sur un mélange de deux précurseurs **(VIc-1),** (**VIc-2**) ou (**VIa-1**), (**VIa-2**), il doit statistiquement se former, dans le cas de dimères cycliques, un hétérodimère **(IIIf2)** et deux homodimères **(IIIfl)** et **(IIIf3)**.

### Exemple 3

### Clivage du dérivé activé d'acide carbamique contenant une fonction amine protégée par rapport au support solide.

Le schéma 3 ci-dessous représente le clivage, par rapport à un support solide (une résine), d'un dérivé activé d'acide carbamique de formule :

B-A-NH-CO-X

dans laquelle
X représente un groupe conférant audit dérivé une fonction d'acide carbamique activé,
B représente la fonction amine protégée,
A représente la partie de la molécule séparant la fonction acide carbamique activé et la fonction amine protégée,
■ représente le support solide (résine).

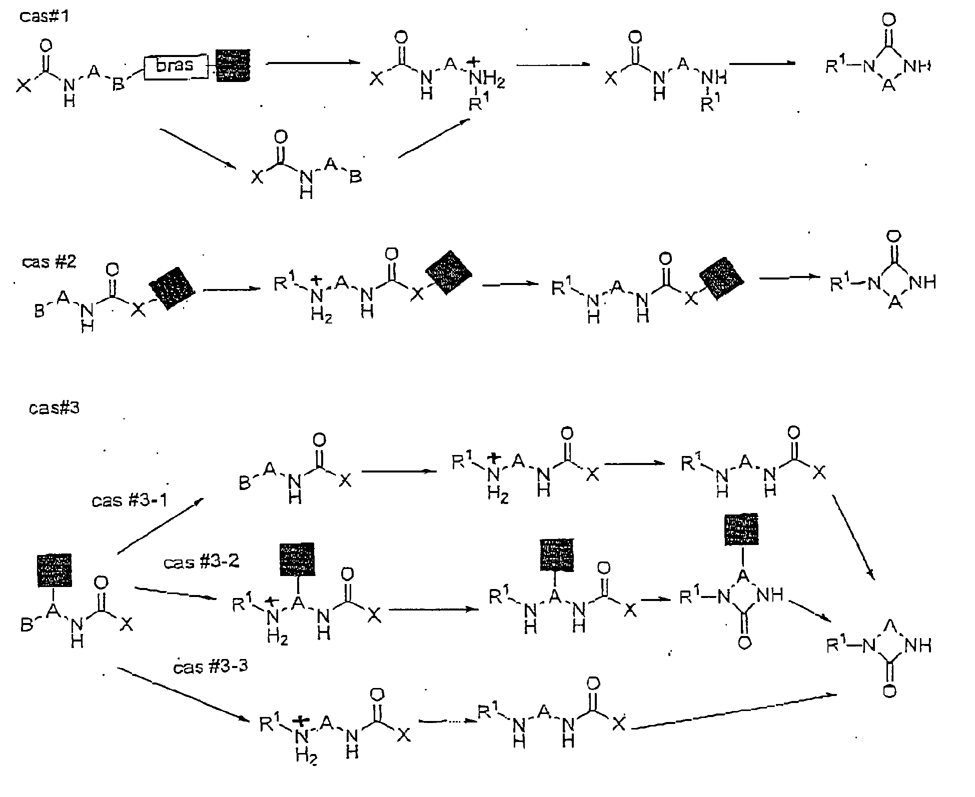

Le dérivé activé d'acide carbamique est lié chimiquement à la résine soit (1) par sa fonction amine (la résine et le bras de la résine servant de groupement protecteur) [cas#1], soit (2) par sa fonction acide carbamique activé [cas#2], soit (3) par un autre groupement fonctionnel présent dans ledit dérivé activé d'acide carbarnique [cas#3].

En fonction du mode d'accrochage du dérivé activé d'acide carbaimque à la résine, le clivage peut être réalisé soit avant l'étape de cyclisation intramoléculaire (l'étape de cyclisation sera donc réalisée en solution après le clivage) [cas#1, cas#2, cas#3-1 et cas#3-3] soit après l'étape de cyclisation (qui aura donc lieu sur le support solide) [cas#3-2].

### Exemple 4

### Procédé de préparation de composés urées cycliques à partir de dérivés d'acide carbamique N-Boc protégés (XVII) et (XIX).

La sélectivité de la déprotection de la fonction amine est dépendante du type de groupement protecteur utilisé pour l'amine et du groupe acide carbamique activé. Un tel exemple d'orthogonalité (à savoir la libération sélective de la fonction amine afin de ne pas altérer la fonction acide carbamique du dérivé activé d'acide carbamique) est montré ci-dessous par la déprotection sélective du groupement Boc (ter-butoxycarbonyle) qui n'altère pas l'intégrité du carbamate de O-succinimidyle (**XVII**), et (**XIX**). En effet, le carbamate de O-succinimidyle n'est pas dégradé en présence d'acide trifluoroacétique ou d'acide chlorhydrique en solvant organique : l'étape de libération de la fonction amine (via la déprotection du groupement Boc) est donc complètement sélective.

Cet exemple n'est en aucun cas limitatif. On peut imaginer d'autres types d'orthogonalité comme l'utilisation du groupement benzyloxycarbonyle avec le carbamate de O-succinimidyle.

**1)** Etape de libération ou de déprotection de la fonction amine protégée des dérivés activés stables d'acide carbamique (**XVII**) et (**XIX**) conduisant respectivement aux dérivés d'acide carbamique contenant une fonction amine primaire ou secondaire sous forme protonée (**XVIIIa**) et (**VIc-3**), ou sous forme libre (**XVIIIb**) ou (**VIa-3**).

Des études ont permis de montrer que le carbamate de N-hydroxysuccinimide était stable en milieu acide (traitement à l'acide trifluoroacétique (TFA) par exemple). Les dérivés d'acides carbamiques de N-hydroxysuccinimide (**XVII**) et (**XIX**) représentés dans le schéma 4 ci-dessous ont été synthétisés comme décrit précédemment, à savoir par :
- une étape de transformation du groupe -COOH du dérivé d'acide aminé N-protégé correspondant en groupe -CON₃ pour obtenir un acyl azide,
- une étape de transformation du groupe -CON₃ de l'acyl azide correspondant en groupe -NCO pour obtenir un isocyanate, une étape de traitement de l'isocyanate correspondant pour obtenir le dérivé d'acide carbamique de N-hydroxysuccinimide de formule (**XVII**) ou (**XIX**).

Les schémas de synthèse des dérivés activés d'acide carbamique de N-hydrosuccinimide (**XVII**) et (**XIX**) sont respectivement représentés ci-dessous :

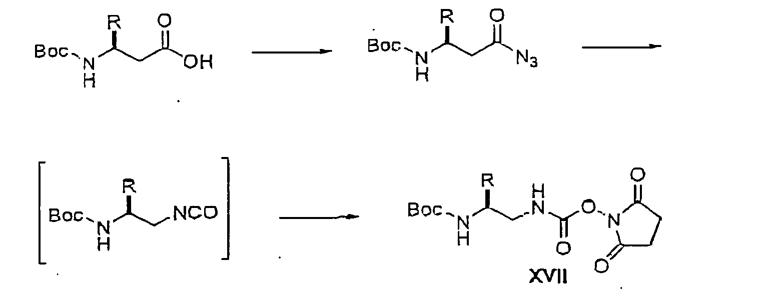

Les conditions expérimentales des différentes étapes d'obtention du dérivé d'acide carbamique (**XVII**) sont décrites dans lapublication de Guichard et al., «J. Org. Chem., 1999, 64, 8702-8705 ».

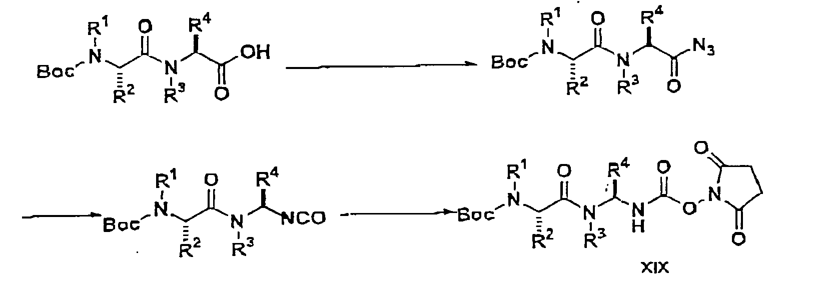

Les conditions expérimentales des différentes étapes d'obtention du dérivé d'acide carbamique (**XIX**) sont identiques à celles décrites pour le composé (**XVII**)**.**

Le schéma 4 ci-dessous représente la déprotection sélective par l'acide trifluoroacétique (TFA) du groupement Boc des dérivés d'acide carbamique (**XVII**) et (**XIX**) de succinimidyle N-protégés.

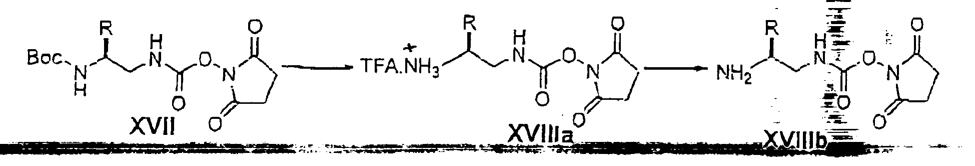

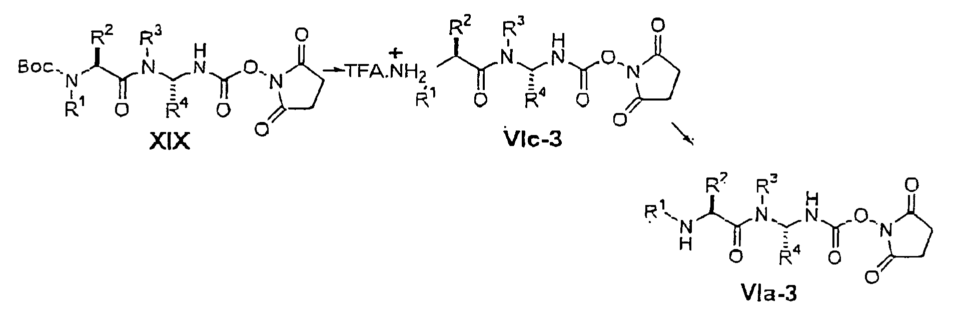

Le traitement des carbamates **(XVII)** et **(XIX)** N-Boc protégés par du TFA permet d'obtenir les composés intermédiaires **(XVIIIa)** et (**VIc-3**) (sels de trifluoroacétate que l'on peut isoler), ou **(XVIIIb)** et **(VIa-3)** (non isolables) qui représentent des précurseurs intéressants pour la synthèse de composés urées cycliques.

Les dérivés d'acide carbamique N-Boc protégés **(XVII)** et **(XIX)** sont dissous chacun dans une solution d'acide trifluoroacétique (CF₃COOH ou TFA) (solvant de déprotection) contenant du chlorure de méthylène (CH₂Cl₂) (50/50 v/v par exemple) ou, dans une solution de TFA pur. Aprés 30 min, le TFA est évaporé ou coévaporé en présence d'éther (Bt₂O) ou d'hexane. Dans un certain nombre de cas, l'ajout d'éther ou d'hexane conduit à un précipité qui est filtré et séché sous vide. Sinon, l'huile résiduelle après évaporation du TFA est séchée sous vide.

2) Etape de cyclisation intramoléculaire des composés précurseurs (**XVIIIb**) et (**VIa-3**).

Les sels de TFA (**XVIIIa**) et (**VIc-3**) précédemment isolés sont chacun dissous ou suspendus dans un volume de solvant (par exemple de l'acétonitrile (MeCN)) (solvant de cyclisation) pour atteindre une dilution allant d'environ 0,0001M à environ 0,1M. Une base tertiaire (au moins un équivalent pour neutraliser le sel de l'amine formé au cours de l'étape de déprotection) par exemple la diisopropyléthylamine, la N-méthylmorpholine, la triéthylamine (Et₃N), la lutidine ou la collidine (pure ou diluée dans un solvant organique tel que MeCN) est ajoutée (soit en goutte à goutte, soit directement) à la solution du sel de trifluoroacélate pendant une période de temps pouvant aller jusqu'à 24 heures.

Les composés **(XVIIIb)** et **(VIa-3)** ainsi obtenus réagissent intramoléculairement pour conduire aux composés urées cycliques correspondants qui possèdent des tailles de cycles différentes.

La réaction est suivie par chromatographie liquide haute performance (HPLC). Lorsque la réaction n'évolue plus, le solvant est évaporé et le résidu est purifié soit par HPLC en phase inverse, soit par chromatographie éclair sur silice, soit par recristallisation dans un solvant approprié pour donner le ou les composés urées cycliques attendus.

Les schémas 5 à 11 ci-dessous représentent respectivement :
- l'étape de libération de la fonction amine protégée d'un dérivé activé stable d'acide carbamique répondant à la formule générale (**XVII**) ou (**XIX**) et,
- l'étape de cyclisation intramoléculaire à partir du dérivé d'acide carbamique ainsi obtenu contenant une fonction amine sous forme libre. 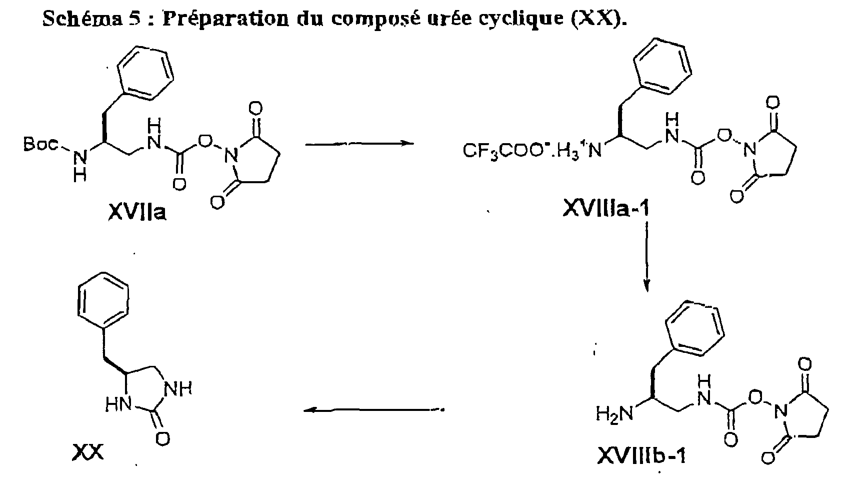
   1) Le composé (**XVIIa**) (323 mg, 0.8 mmol) est dissous dans 10 ml d'un mélange TFA/CH₂Cl₂ (50/50 v/v), et la solulton est agitée à température ambiante pendant 30 minutes. Le solvant est ensuite concentré et le sel de TFA (**XVIIIa-1**) est précipité par ajout d'hexane. Le précipité est filtré et séché à la pompe à palette pendant 12h pour donner un solide blanc (**XVIIIa-1**) (300 mg, 93%).
   2) Le composé (**XVIIIa-1**) (300 mg, 0.72 mmol) est dissous dans MeCN (10 ml) et une solution de diisopropyléthylamine (130 µl, 0.73 mmol) est ajoutée. Le mélange réactionnel est agité pendant 60 minutes. MeCN est évaporé et le résidu est redissous avec de l'acétate d'éthyle. La phase organique est lavée avec une solution saturée de NaCl pour donner le composé (**XX**) (110 mg, 87%).

Dans le cas du dérivé d'acide carbamique (**XVIIa**) (dérivé d'acide β-aminé), le composé cyclique (**XX**) obtenu après déprotection du groupement Boc et cyclisation intramoléculaire, comporte un cycle à 5 atomes. Ce composé a été précédemment décrit dans la littérature.

Cet exemple a été donné afin de montrer que le procédé de préparation de la présente invention permet d'obtenir des composés urées cycliques déjà décrits dans la littérature. 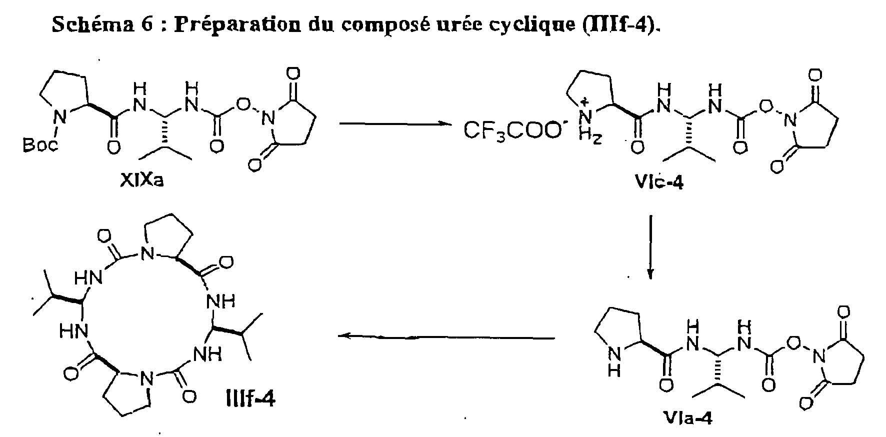
**1)** Le composé (**XIXa**) (500 mg, 1.17 mmol) est dissous dans 10 ml d'un mélange TFA/CH₂Cl₂ (50/50 v/v), et la solution est agitée à température ambiante pendant 30 minutes. Le solvant est ensuite concentré et le sel de TFA (**VIc-4**) est précipité par ajout d'étber. Le précipité est filtré et séché à la pompe à palette pendant 12h pour donner un solide blanc (**VIc-4**) (450 mg, 87%).
**2)** Le composé (**VIc-4**) (430 mg, 0.97 mmol) est dissous dans 80 ml MeCN et la solution est ajoutée goutte à goutte à une solution de diisopropyléthylamine (421 µl, 2.4 mmol) dans MeCN (500 ml) pendant 1h. Le mélange réactionnel est agité pendant 5h. MeCN est évaporé et le résidu est repris dans CH₂Cl₂. La phase organique est lavée avec KHSO₄ 1N, séchée sur MgSO_{d}, et concentrée. Le résidu est purifié par chromatographic phase inverse sur colonne C18 pour donner le composé (**IIIf-4**) (140 mg, 70%).

Dans le cas du dérivé d'acide carbamique (**XIXa**), dérivé de dipeptide pour lequel la liaison amide n'est pas en configuration cis, on obtient principalement un dimère cyclique (**IIIf-4**) à 14 atomes avec un rendement de 70% (schéma 6).

Le composé (**IIIf-4**) est nouveau.

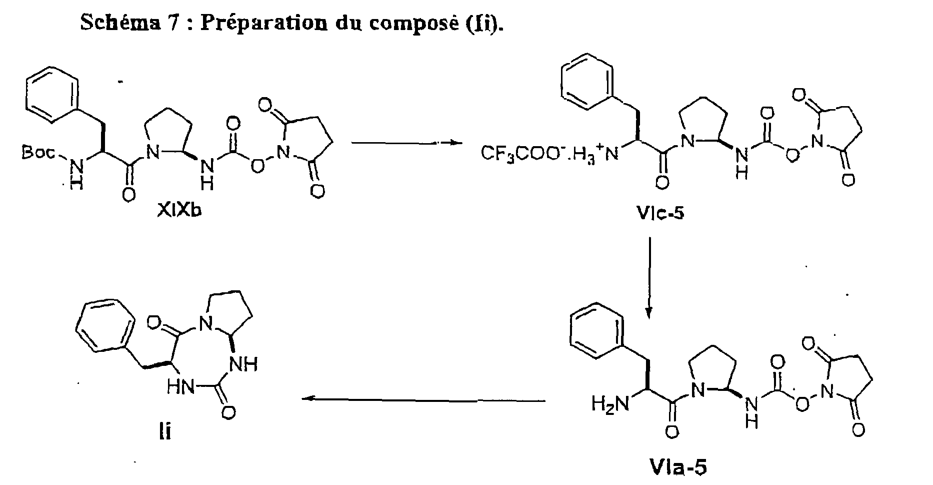

Dans le cas du dérivé d'acide carbamique **(XIXb),** pour lequel la liaison amide peut adopter une configuration cis, on obtient le monomère correspondant cyclique **(Ii)** à 7 atomes avec un rendement supérieur à 70% (schéma 7).

Le composé (**Ii)** est nouveau. 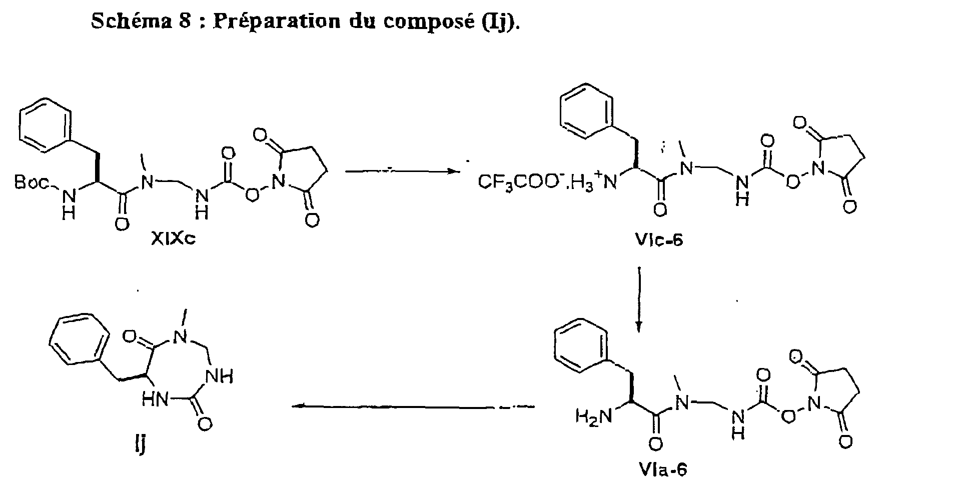
1) Le composé (**XIXc**) (3g, 6.91 mmol) est dissous dans 20 ml d'un mélange TFA/CH₂Cl₂ (50/50 v/v), et la solution est agitée à température ambiante pendant 30 minutes. Le solvant est ensuite concentré et le sel de TFA **(VIc-6)** séché à la pompe à palette pendant 12h pour donner une mousse solide **(VIc-6)** (3.23 g, 100%).
2) Le composé **(VIc-6)** (400 mg, 0.89 mmol) est dissous dans MeCN (30 ml) et la solution est ajoutée goutte à goutte à une solution de diisopropyléthylamine (353 µl, 2.0 mmol) dans MeCN (40 ml) à -20°C pendant 1h. Le melange réactionnel est agité pendant 3h. MeCN est évaporé et le résidu est recristallisé dans un mélange CH₂Cl₂/diisopropyléther pour donner **(Ij)** (135 mg, 70%)

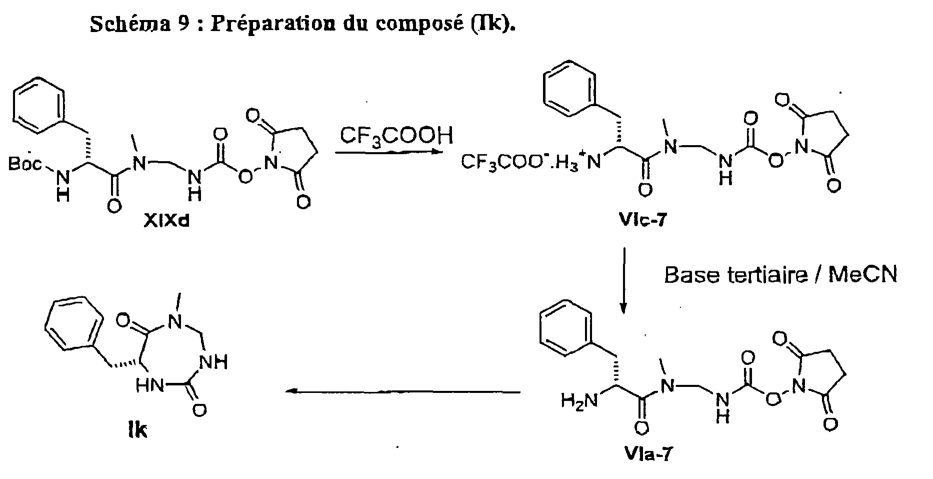

**1)** Le composé **(XIXd)** (2.02g, 4.49 mmol) est dissous dans de l'acide trifluoroacétique (v = 10 ml) pendant 30 minutes. Le précipité qui se forme par ajout de diéthyléther est collecté sur fritté, lavé à l'éther et séché à la pompe à palette pendant 12h pour donner un solide blanc **(VIc-7) :** 1.98g, 95%.
**2)** Le composé **(VIc-7)** (1.94g, 4.17 mmol) est dissous dans MeCN (90 ml) et la solution est ajoutée goutte à goutte. à une solution de diisopropyléthyl amine (1.78 ml, 10.42 mmol) dans MeCN (50 ml) à température ambiante pendant 4h. MeCN est évaporé et le résidu est recristallisé dans un mélange CH₂Cl₂/diisopropyléther pour donner **(Ik)** (586 mg, 60%).

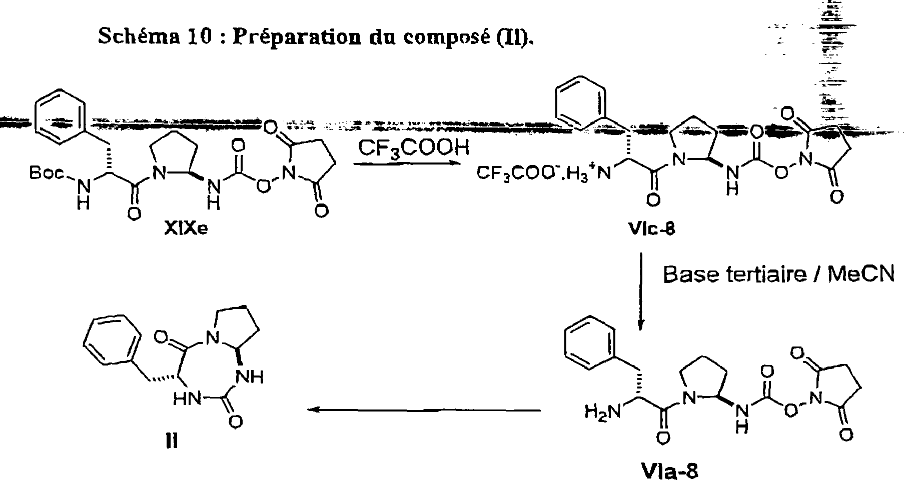

**1)** Le composé **(XIXe)** (1.21g, 2.55 mmol) est dissous dans de l'acide trifluoroacétique (v = 10 ml) pendant 30 minutes. Le précipité qui se forme par ajout de diéthyl éther est collecté sur fritté, lavé à l'éther et séché à la pompe à palette pendant 12h pour donner un solide blanc **(VIe-8)** : (816 mg, 65%).
**2)** Le composé **(VIe-8)** (200 mg, 0.41 mmol) est dissous dans MeCN (20 ml) et la solution est ajoutée goutte à goutte à une solution de diisopropyléthyl amine (0.21 ml, 1.23 mmol) dans MeCN (100 ml) à température ambiante pendant 4h. MeCN est évaporé et le résidu est purifié par HPLC préparative pour donner (**II**) après lyophilisation (70 mg, 66%).

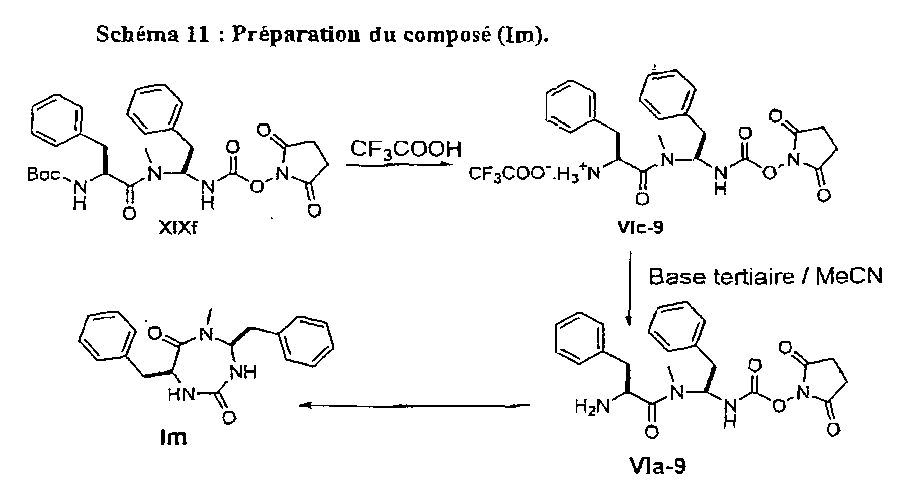

**1)** Le composé **(XTXf)** (1.05g, 1.95 mmol) est dissous dans de l'acide trifluoroacétique (v = 10 ml) pendant 30 minutes. Le précipité qui se forme par ajout de diéthyl éther est collecté sur fritté, lavé à l'éther et séché à la pompe à palette pendant 12h pour donner un solide blanc **(VIc-9)** : (1.045 mg, 97%).
**2)** Le composé **(VIc-9)** (200 mg, 0.36 mmol) est dissous dans MeCN (20 ml) et la solution est ajoutée goutte à goutte à une solution de diisopropyléthyl amine (0.19 ml, 1.08 mmol) dans MeCN (100 ml) à température ambiante pendant 4h. MeCN est évaporé et CH₂Cl₂ (1.5 ml) est ajouté. Le dérivé urée cyclique est alors purifié par traitement avec une résine "scavenger" (Tris-(2-aminoéthyl)-amine polystyrène) pour donner (**Im**) : (116 mg, 99%).

**Tableau 1** : composés urées cycliques (**XX**), (**IIIf-4**), (**Ti**), (**Ij**), (**Ik**), (**Il**) et (**Im**) obtenus respectivement à partir des dérivés activés stables d'acide carbamique (**XVIIa**), (**XIXa**), (**XIXb**), (**XIXc**), (**XIXd**), (**XIXe**) et (**XIXf**).
^{*a*} rendements des composés urées cycliques (**XX**), (**IIIf-4**), (**Ii**), (**Ij**), (**Ik**), (**Il**) et (**Im**)
^{*b*} gradient linéaire de **A** (solution d'eau contenant 0.1 % TFA) et **B** (solution d'acétonitrile contenant 0.08% TFA),
^{*c*} 5-65% B, 20 min (on passe de 5% à 65% de B en 20 min),
^{d} 0-100% B, 20 min.
HPLC : chromatographie liquide haute performance
MALDI-MS : spectrométrie de masse

| Carbamates (**XVII**) ou (**XTX**) | Urées cycliques | Rendement (%)^{*a*} | HPLC *t*_{R}(min)^{*b*} | MALDI-MS |
|---|---|---|---|---|
| (**XVIIa**) | (**XX**) | 90 | 11,2^{c} | 177.2 [M+H]⁺ |
| (**XIXa**) | (**IIIf-4**) | 70 | 10.00^{d} | 367.4 [M+H]⁺ |
| (**XIXb**) | (**Ii**) | 70 | 10.03^{d} | 260.3 [M+H]⁺ |
| (**XIXc**) | (**Ij**) | 70 | 9,39^{d} | 234.5 [M+H]⁺ |
| (**XIXd**) | (**Ik**) | 60 | 9,22^{d} | 243.3 [M+H]⁺ |
| (**XIXe**) | (**Il**) | 66 | 9,55^{d} | 260.4 [M+H]⁺ |
| (**XIXf**) | (**Im**) | 99 | 7,88^{d} | 324.2 [M+H]⁺ |

Les données physico-chimiques des composés (**IIIf-4), (Ii), (Ij)** et **(Ik)** sont données ci-dessous.
**(IIIf-4)** : Rendement 70 % solidc blanc ; HPLC *t*_{*r*} 10.0 min (gradient linéaire, 0-100% B, 20 min) - ¹H RMN ([D₆]DMSO, 200 MHz) : δ = 0.86 (d, *J =* 6.8 Hz, 6H, Me), 0.86 (d, *J* = 6.7 Hz, 6H, Me), 1.59-1.70 (m, 2H, C*H*(Me)₂), 1.76-2.11 (m, 1H, CHC*H*₂C*H*₂), 3.14-3.26 (m, 1H, C*H*₂N), 3.49-3.60 (m, 1H, C*H*₂N), 4.80 (m, 1H, NHC*H*NH), 5.58 (d, *J* = 8.9 Hz, NCONH), 6.48 (d, *J=* 6.5 Hz, CH₂CON*H*).
**(Ii) :** Rendement 70 % solide blanc ; HPLC *t*_{*r*} 10.0 min (gradient linéaire, 0-100% B, 20 min) - ¹H RMN ([D₆]DMSO, 200 MHz) : δ = 1.89-2.29 (m, 4H, CHC*H*₂C*H*₂), 2.78 (dd, *J* = 8.7, 14.5 Hz, 1H, CH₂Ph), 3.37 (dd, *J* = 5.4, 14.4 Hz, 1H, CH₂Ph), 3.45-3.55 (m, 1H, CH₂N), 3.75-3.86 (m, 1H, CH₂N), 4.59 (hept, J = 2.7, 5.6, 8.5), 4.84 (s, 1H, NH), 5.46 (br q, *J* = , 3.3 Hz, 1H, NC*H*NH), 6.4 (s, 1H, NH), 7.20-7.35 (m, 5 arom. H).
**(Ij)** : Rendement 80% solide blanc ; HPLC *t*_{*r*} 9.39 min (gradient linéaire, 0-100% B, 20 min) - ¹H RMN ([D₆]DMSO, 400 MHz) : δ = 6.18 (s, H, NHPhe, 1H), 5.17 (d, ^{α}CH-*gem*-Sar , 1H), 4.77 (m, ^{α}CH Phe, 1H), 4.10 (dd, ^{α}CH-*gem*-Sar , 1H).
**(Ik)** : Rendement 90% solide blanc ; HPLC *t*_{*r*} 9.22 min (gradient linéaire, 0-100% B, 20 min) - ¹H RMN ([D₆]DMSO, 400 MHz) : δ = 6.18 (s, H, NHPhe, 1H), 5.17 (d, ^{α}CH-*gem*-Sar , 1 H), 4.77 (m, ^{α}CH Phe, 1 H), 4.10 (dd, ^{α}CH-*gem*-Sar, 1H).

### Exemple 5

### Préparation de composés urées cycliques par mono- ou di-alkylation des composés urées cycliques (Ij)

### 1) Préparation des composés (In), (Io), (Ip) et (Iq) par dialkylation de (Ij)

*Méthode générale :* à une solution de **(Ij)** (1 équivalent) dans du THF distillé est ajouté NaH (5 équivalents) puis l'électrophile (RX, 3 équivalents). Le mélange réactionnel est laissé sous agitation pendant 3 à 48b. La réaction est suivie par RP-HPLC sur colonne C18. A la fin de la réaction, de l'acétate d'éthyle est ajouté et la phase organique est lavée avec du NH₄Cl. Afin d'éliminer l'excès d'électrophile si celui ci n'est pas volatile, et si l'on ne désire pas faire de purification sur silice, on peut utiliser une résine "scavenger", comme la résine (mercaptoéthyl)aminoéthylpolystyrène par exemple. Dans ce cas, on ajoute la résine "scavenger" (environ 10 équivalents) au milieu réactionnel et on laisse le mélange sous agitation pendant 48h. A l'issue de ce traitement, la résine est éliminée par filtration et la phase organique est lavée avec NH₄Cl, séchée et concentrée pour donner, le produit désiré purifié comme cela est montré sur le schéma 12.

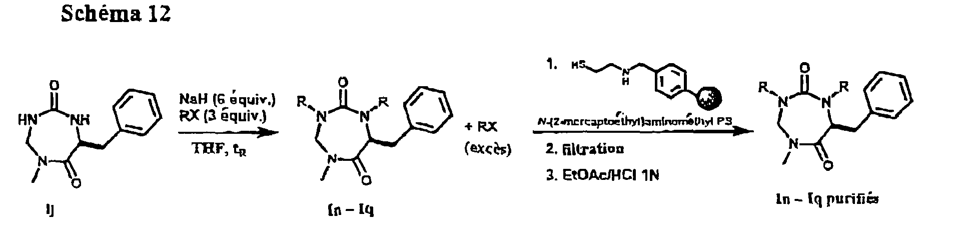

Pour les composés (**In**), (**Io**), (**Ip**) et (**Iq**), le groupe R représente respectivement : un groupe méthyle (Me), un groupe -CH₂COOtBu, un groupe Bn (-CH₂-Φ) et un groupe BnOBn.

**Tableau 2**

| Produit | RX | Résine "scavenger" | Pureté (%) | Rendement (%) | HPLC *t*_{R} (min) ^{a} |
|---|---|---|---|---|---|
| In | MeI | non | 95 | 99 | 10.66 ^{b} |
| Io | BrCH₂COOtBu | oui | 94 | 95 | 15.82 ^{b} |
| Ip | BnBr | oui | 94 | 90 | 16.30 ^{b} |
| Iq | BnOBnBr + NaI | oui | 86 | 96 | 16.44 ^{c} |

| | | | | | |
|---|---|---|---|---|---|
| ^{*a*} gradient de *A* (0.1% TFA dans H₂O) et *B* (MeCN contenant 0.08% TFA). | | | | | |
| ^{*b*} 0-100% *B*, 20 min. | | | | | |
| ^{*c*} 30-100% *B*, 20 min. | | | | | |

### 2) Préparation des composés (Ir), (Is), et (It) par monoalkylation de (Ij)

*Méthode générale :* à une solution de **(Ij)** (1 équivalent) dans un solvant anhydre (THF, MeCN ou CH₂Cl₂) distillé est ajouté du fluorure de potassium sur alumine (40w/w) (10 équivalents) suivi de l'électrophile (RX, entre 1 et 20 équivalents). Le mélange réactionnel est laissé sous agitation pendant 20-72h. La réaction est suivie par RP-HPLC sur colonne C18. A la fin de la réaction, le fluorure de potassium sur alumine est éliminé par filtration. Afin d'éliminer l'excès d'électrophile si celui ci n'est pas volatile, et si l'on ne désire pas faire de purification sur silice, on peut utiliser une résine "scavenger", comme la résine (mercaptoéthyl)aminoéthylpolystyrène par exemple. Dans ce cas on ajoute la résine "scavenger" (environ 10 équivalents) au milieu réactionnel et on laisse le mélange sous agitation pendant 48h. A l'issue de ce traitement, la résine est éliminée par filtration et la phase organique est concentrée pour donner le produit désiré purifié comme cela est montré sur le schéma 13 et dans le tableau 3. Il est possible dans les meilleures conditions (environ 1 équivalent de RX, temps de réaction de 48h, voir tableau 3) d'obtenir une sélectivité de produit mono-alkylé par rapport au produit di-alkylé de l'ordre de 93:7.

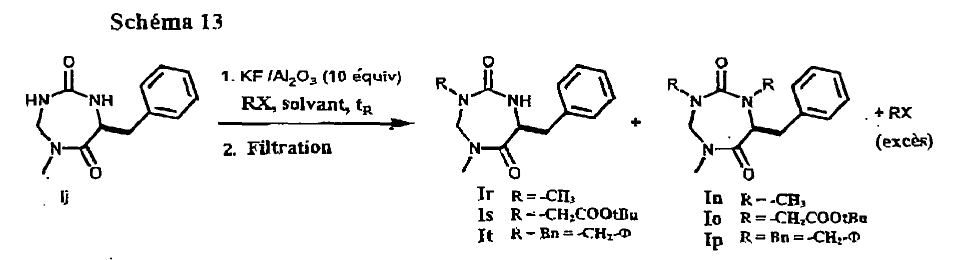

**Tableau 3**

| RX | eq. | solvant | temps (heures) | Mono- Di-alkylé | Pureté globale (mono + di) | HPLC t_{R} (mono/di) (min)^{d} |
|---|---|---|---|---|---|---|
| McI | 1.05 | THF | 48h | 93:7 | 70^{c} | 10.08/10.65 |
| McI | 20 | MeCN | 72h | 10:90 | 92 | |
| BrCH₂COOtBu | 1.0 | THF | 48 | 93:7 | 87^{k,o} | 12.73/15.79 |
| BrCH₂COOtBu | 1.5 | THF | 48 | 93:7 | 93^{a} | |
| BrCH₂COOtBu | 10 | MeCN | 20 | 89:11 | 67^{a} | |
| BnBr | 2 | DMF | 20 | Réaction difficile | - | 13.18/16.17 |
| BnBr | 2 | THF | 72 | 72:28 | 96^{a} | |
| BnBr | 1.05 | THF | 48 | 87:13 | 87^{a} | |
| BnBr | 1.05 | MeCN | 72 | 83:17 | 87^{a} | |
| BnBr | 1 | CH₂Cl₂ | 48 | 94:6 | 74^{a,b} | |

| | | | | | | |
|---|---|---|---|---|---|---|
| ^{a} après traitement avec la résine N-(2-mercaptoéthyl)aminométhylpolystyrène. | | | | | | |
| ^{b} 6% de produit de départ Ij est présent. | | | | | | |
| ^{c} 20% de produit de départ Ij est présent. | | | | | | |
| ^{d} 10% de produit de départ Ij est présent. | | | | | | |
| ^{e} gradient de A (0.1% TFA dans H₂O) et B (McCN contenant 0.08% TFA). 0-100% B, 20 min. | | | | | | |

### Exemple 6

### Propriétés structurales des composés urées cycliques (I)

Les composés urées cycliques (**I**) ont une structure extrêmement contrainte, bien définie sur la base de la diffraction des rayons X ou bien de la RMN. La connaissance de cette structure est extrêmement utile pour l'utilisation de la plate-forme hétérocyclique basée sur les composés (**I**) pour la conception et la découverte de nouveaux composés d'intérêt pharmacologique. La structure de **(Ij)** (voir Figures 1A et 1B) a été obtenue par diffraction des rayons X et est représentative de la structure des composés (**I**). Cette structure est en accord avec celle obtenue pour le même composé par RMN bidimensionnelle et par modélisation. Le cycle 1,3,5-triazépin-2,6-dione a une conformation fortement repliée. Les plans des deux groupes amide et urée se rencontrent le long d'une ligne rejoignant le carbone en alpha du résidu gem-Sarcosine (-N(CH₃)-^{α}CH₂-NH-) et le carbone alpha de la phénylalanine avec un angle dièdre de 120". A titre de comparaison, dans le cas des dicétopipérazines les plus repliées, l'angle dièdre défini par les plans amide est de l'ordre de 140-160°. Par ailleurs, les atomes d'hydrogène en positions axiales sur le carbone en alpha du résidu gem-Sarcosine et le carbone alpha de la phénylalanine sont extrêmement proches dans l'espace : ils ne sont séparés que par 2.03 angströms. A titre de comparaison, la distance entre les protons situés sur les carbones en alpha dans les dicétopipérazines repliées est de l'ordre de 2.7-2.8 angströms.

### Exemple 7

### Préparation de composés (XV) et (XVIa) selon les schémas 14 à 20.

La séquence de réaction conduisant à l'obtention du composé (XVIa-2) est représentée sur le schéma 14 et la procédure détaillée est donnée ci-dessous.

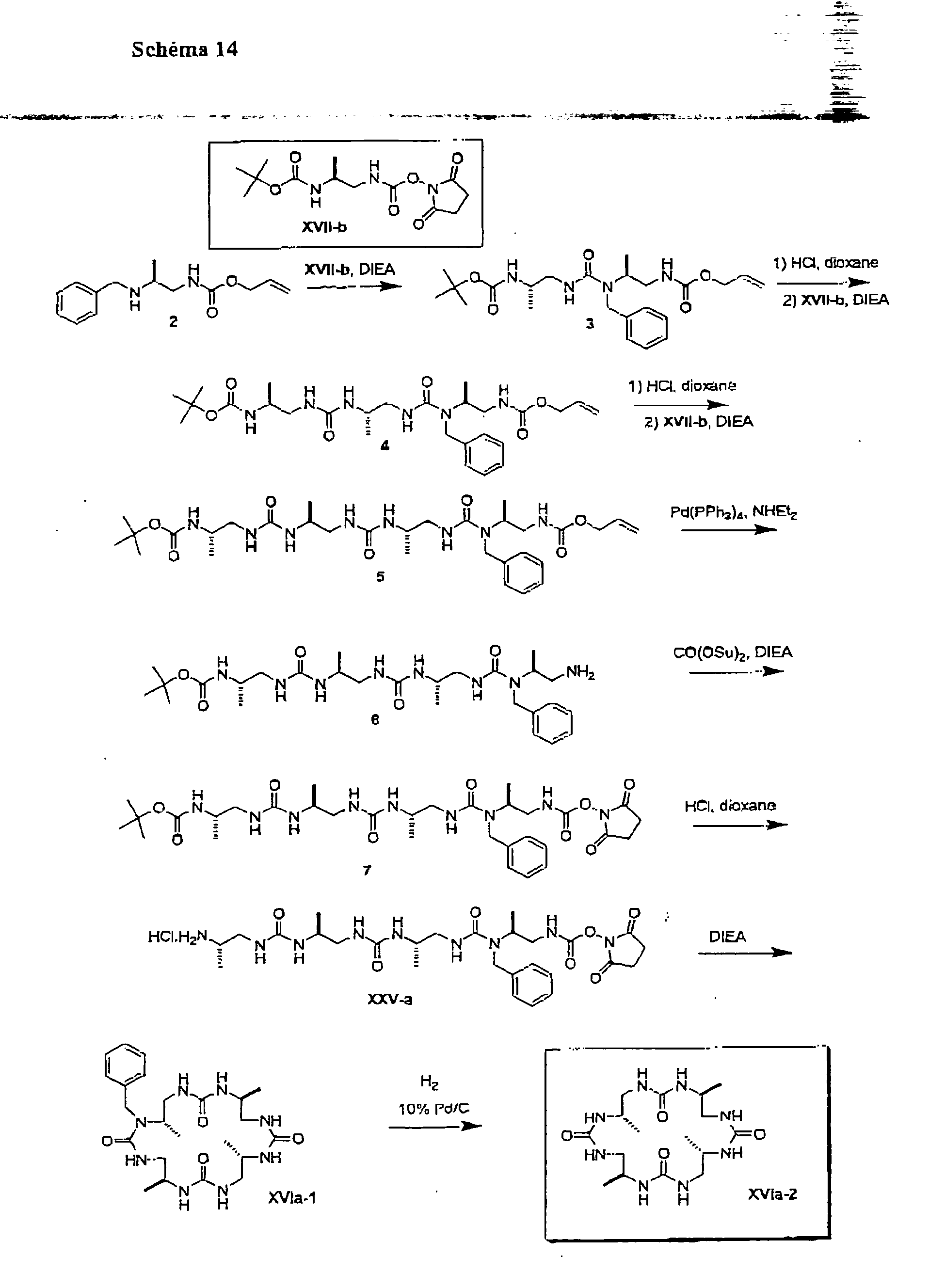

A une solution du composé 2 (voir formule ci-dessus, dans le schéma 14) (930 mg ; 3,74.10⁻³ mol) dans de l'acétonitrile (5 ml), on ajoute la DIEA (1,27 ml : 7,48.10⁻³ mol) puis le composé (**XVII-b**) (1,18 g; 3,74.10⁻³ mol). La réaction est suivie par CCM. Au bout de 30 min, l'acétonitrile est évaporé et le résidu repris dans l'acétate d'éthyle, lavé consécutivement par des solutions 1N KHSO₄, saturée NaHCO₃, et enfin saturée NaCl. La phase organique ainsi obtenue est séchée (Na₂SO4) et évaporée. Le résidu est chromatographié sur silice avec un système AcOEt/Hexane 50/50, afin d'obtenir le composé **3**. Rendement 74% (1.26 g). Huile translucide. HPLC *t*_{*R*} 14,89 min (gradient linéaire, 20-80 B, 20 min).

Le composé **3** (650 mg, 1,45.10⁻³ mol) est déprotégé par adjonction d'acide trifluoroacétique (3 ml) sous agitation. Ce dernier est éliminé par coévaporations successives à l'aide d'hexane jusqu'à obtenir un résidu que l'on sèche. Le produit ainsi obtenu est solubilisé dans de l'acétonitrile (5 ml). On additionne la DIEA (246 µl ; 1,45.10⁻³ mol) puis le composé (**XVII-b**) (457 mg; 1,45.10⁻³ mol). La réaction est suivie par CCM. Au bout de 30 min, l'acétonitrile est évaporé et le résidu repris dans l'acétate d'éthyle, lavé consécutivement par des solutions 1N KHSO₄, saturée NaHCO₃, et enfin saturée NaCl. La phase organique ainsi obtenue est séchée (Na₂SO4) et évaporée, afin d'obtenir le composé 4. Rendement 87% (700 mg). HPLC *t*_{*R*} 13,72 min (gradient linéaire, 20-80 B, 20 min) ; MS (MALDI-TOF) m/z 548,46 [M + H]⁺, 571,31 [M + Na]⁺, 587,81 [M + K] ⁺.

Le composé 4 (640 mg; 1,17.10⁻³ mol) est déprotégé par adjonction d'acide trifluoroacétique (3 ml) sous agitation. Ce dernier est éliminé par coévaporations successives à l'aide d'hexane jusqu'à obtenir un résidu que l'on sèche. Le produit ainsi obtenu est solubilisé dans de l'acétonitrile (5 ml). On additionne la DIEA (199 µl ; 1,17.10⁻³ mol) puis le composé (**XVII-b**) (368 mg ; 1,17.10⁻³ mol). La réaction est suivie par CCM. Au bout de 30 min, l'acétonitrile est évaporé et le résidu repris dans l'acétate d'éthyle, lavé consécutivement par des solutions IN KHSO₄, saturée NaHCO₃, et enfin saturée NaCl. La phase organique ainsi obtenue est séchée (Na₂SO4) et évaporée, afin d'obtenir le composé 5. Rendement 70% (530 mg). HPLC *t*_{*R*} 13.62 min (gradient linéaire, 20-80 B, 20 min); MS (MALDI-TOF) m/z 671.17 [M + Na]⁺, 687.68 [M + K]⁺.

A une solution du composé 5 (460 mg ; 7,09.10⁻⁴ mol) dans du dichlorométhane (5 ml), on ajoute 1% mol Pd(PPh₃)₄ (8,2 mg ; 7,09.10⁻⁶ mol) suivi de NHEt₂ (600 µl ; 4,25.10⁻³ mol). La réaction est suivie par CCM. Au bout de 30 min, le dichlorométhane est évaporé. Le résidu est repris dans de l'eau (50 ml) à 1% d'acide acétique et lavé avec AcOEt à deux reprises puis lyophilisé, afin d'obtenir le composé 6**.** Rendement quantitatif (446 mg). HPLC *t*_{*R*} 7.40 min (gradient linéaire, 20-100 B, 20 min); MS (MALDI-TOF) m/z 565.68 [M + H]⁺, 587.38 [M + Na]⁺.

On prépare une solution du composé 6 (75.5 mg; 1,34.10⁻³ mol) dans de l'acétonitrile (400 µl). Parallèlement, on prépare une solution de carbonate de succinimidyle (69 mg ; 2,68.10⁻³ mol) dans 2,69 ml d'acétonitrile. On additionne à 8 reprises 50 ml de la solution de carbonate de succinimidyle dans la solution de **6,** suivie de l'addition d'environ 6 µl de DIEA. Après la dernière adjonction, le mélange est laissé sous agitation pendant une heure et est ensuite évaporé. On obtient alors le composé 7. HPLC τ_{*R*} 11,32 min (gradient linéaire, 20-80 B, 20 min) ; MS (MALDI-TOF) m/z 729,03 [M + Na]⁺, 745,31 [M + K]⁺.

Dans un premier temps, on déprotège le composé **7** à l'aide d'HCl dans le dioxane pour donner **(XXV-a).** Le mélange est coévaporé plusieurs fois et le résidu séché. Ensuite, à une solution de DIEA (442 µl ; 2,68.10⁻³ mol) dans 100 ml d'acétonitrile est ajouté goutte à goutte pendant une heure une solution du produit **(XXV-a)** précédemment obtenu dans 10 ml d'acétonitrile. Le mélange est évaporé et purifié par HPLC préparative (gradient linéaire 0-80) pour donner **(XVIa-1).** HPLC *t*_{*R*} 7.56 min (gradient linéaire, 20-80 B, 20 min) ; MS (MALDI-TOF) m/z 491.02 [M + H]⁺, 513.46 [M + Na]⁺ , 529.66 [M + K] ⁺.

A une solution de **(XVIa-1)** (42 mg, 8.57.10⁻³ mol )dans de l'éthanol (10 ml), on ajoute du palladium sur charbon. Après deux heures de réaction sous 1 atm de H₂, on filtre sur Celite® et on évapore. L'agrégat est repris dans 1 ml de TFA jusqu'à la solubilisation partielle, puis on ajoute environ 20 ml d'eau. Le tout est centrifugé et le surnageant lyoghylisé. On obtient alors le composé (**XVIa-2**). Rendement 73% (25 mg). Poudre blanche. HPLC *t*_{*R*} 9.34 min (gradient linéaire, 0-100 B, 20 min) ; MS (MALDI-TOF) m/z 401.19 [M + H]⁺; ¹H RMN (500 MHz, CDCl₃ + TFA, 333 K) δ 1.22 (d, 12H), 3.07 (br s, 4H), 3.39 (br d, 4 H), 3.91 (br s, 4H).

De la même manière, les composés (XV-1) et (XV-2), (XVIa-3) et (XVIa-4), (XVIa-5) et (XVIa-6), (XV-3) et (XV-4), (XV-5), (XV-6) et (XV-7), (XV-8) et (XV-9) sont synthétisés comme indiqué respectivement sur les schémas 15 à 20.

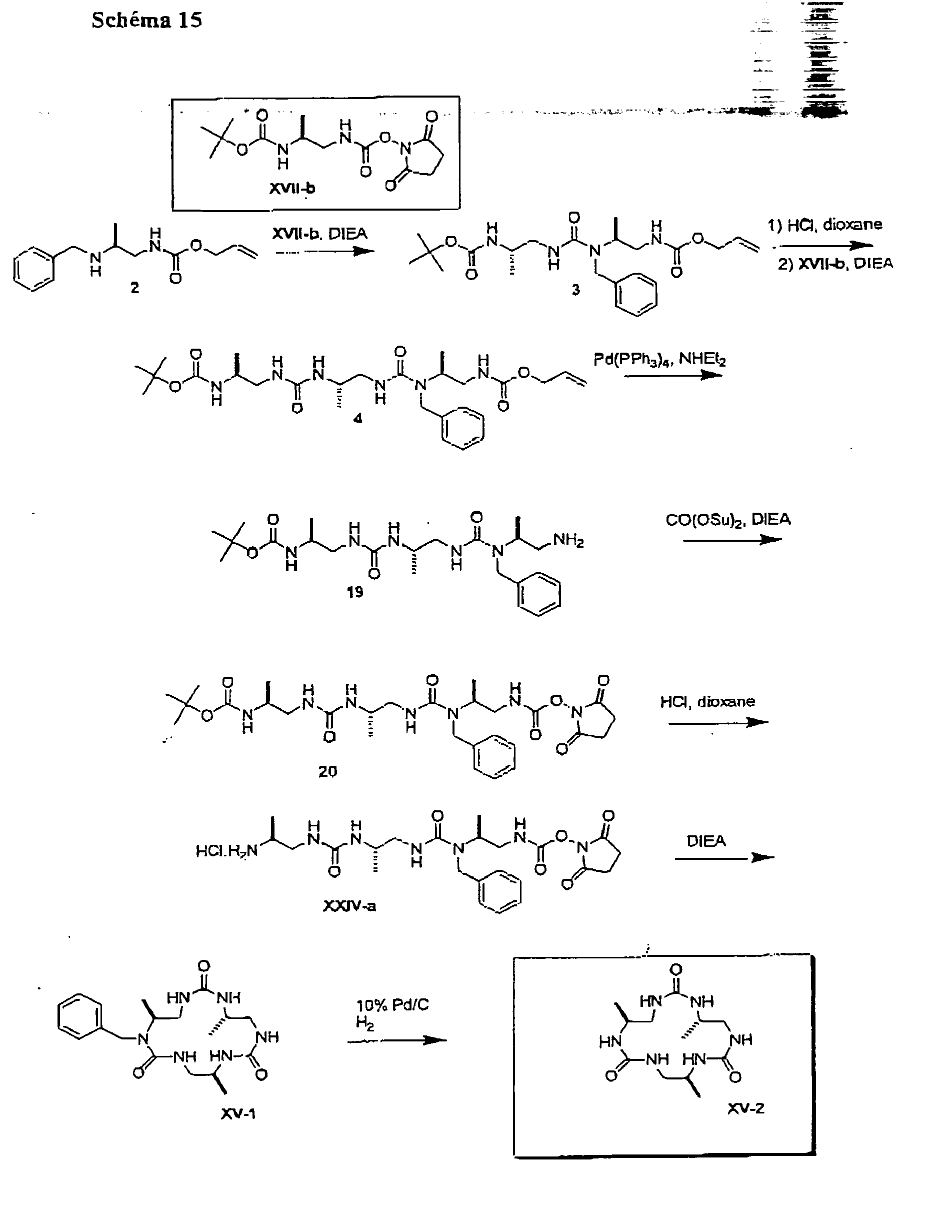

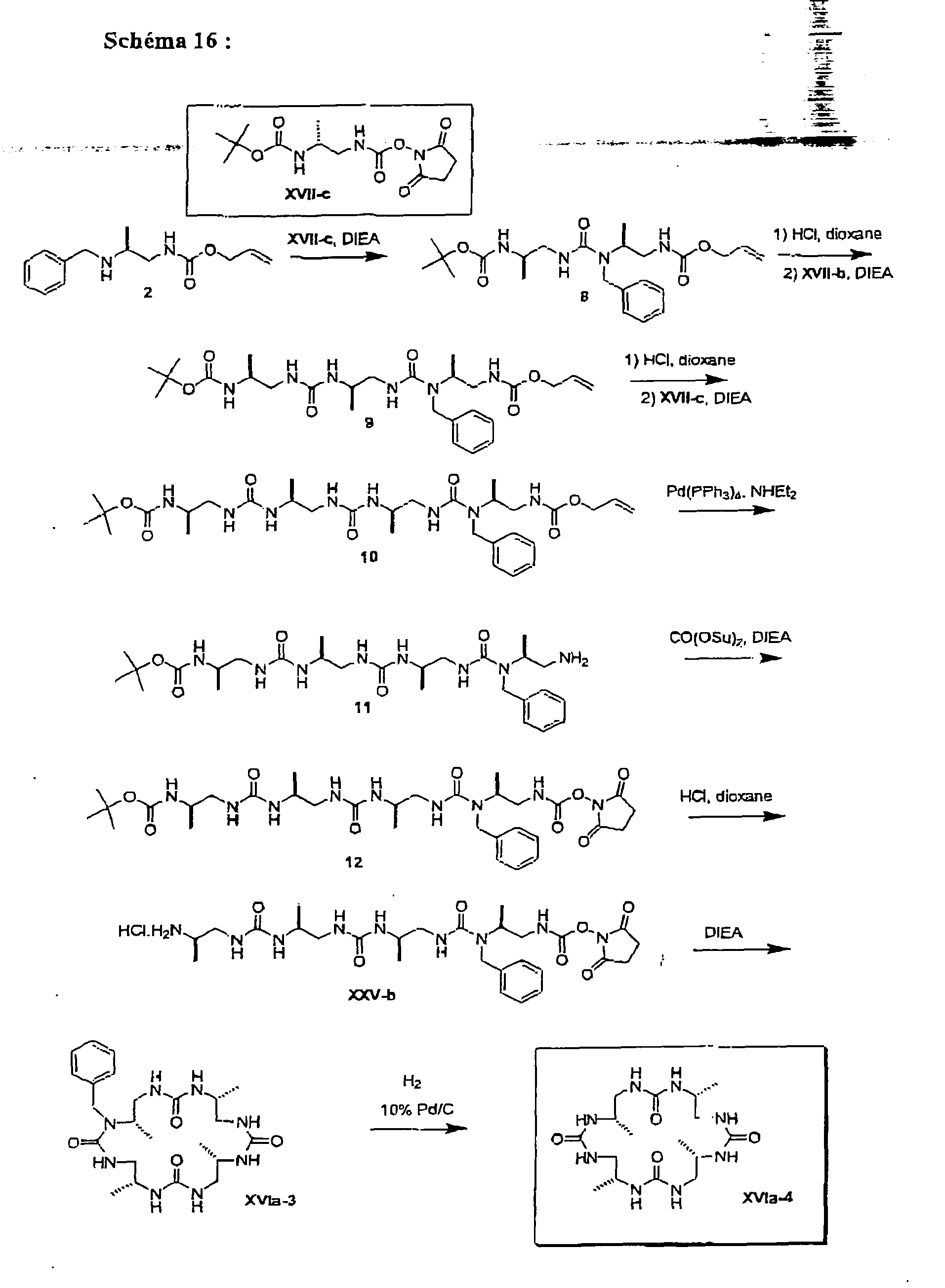

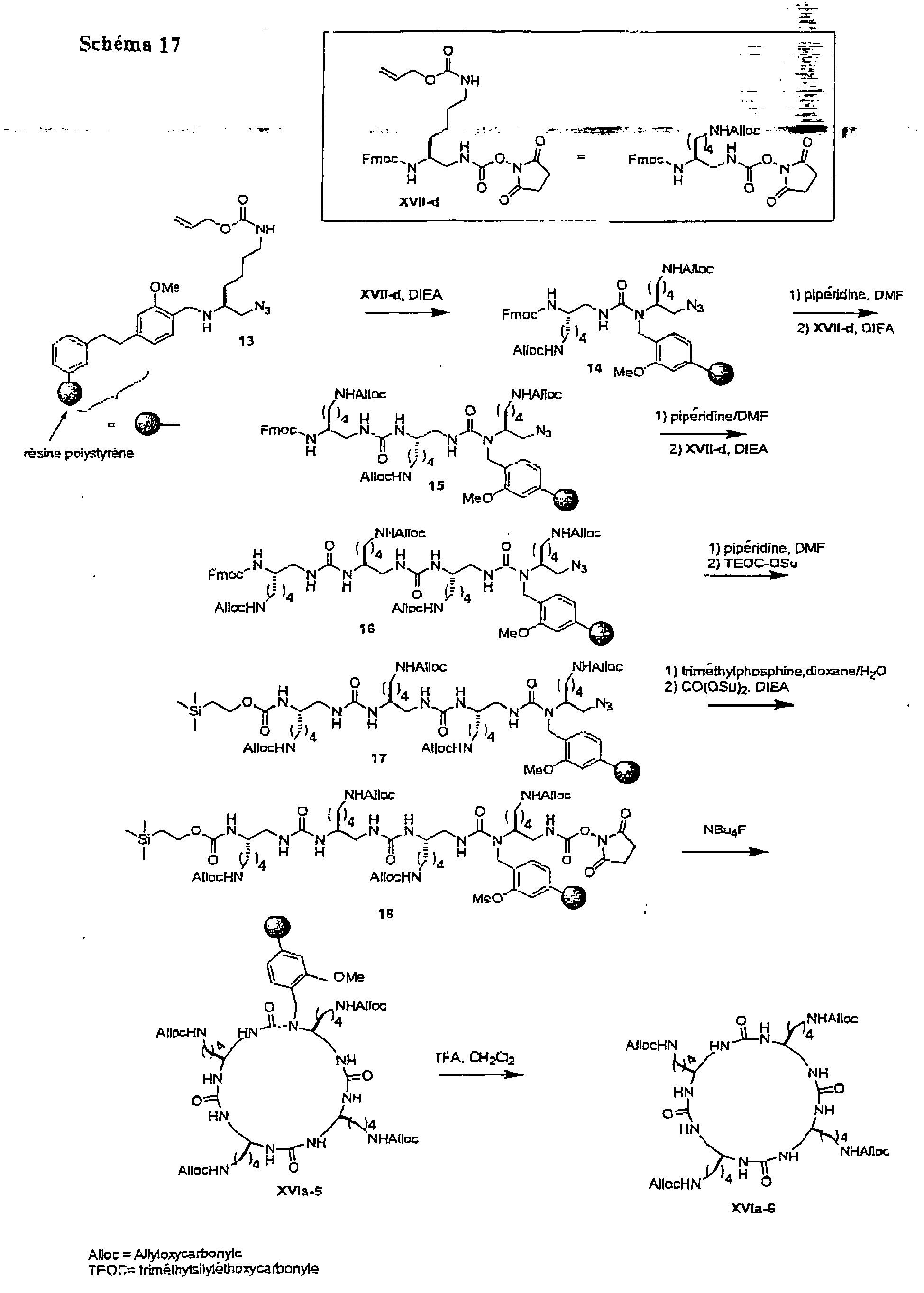

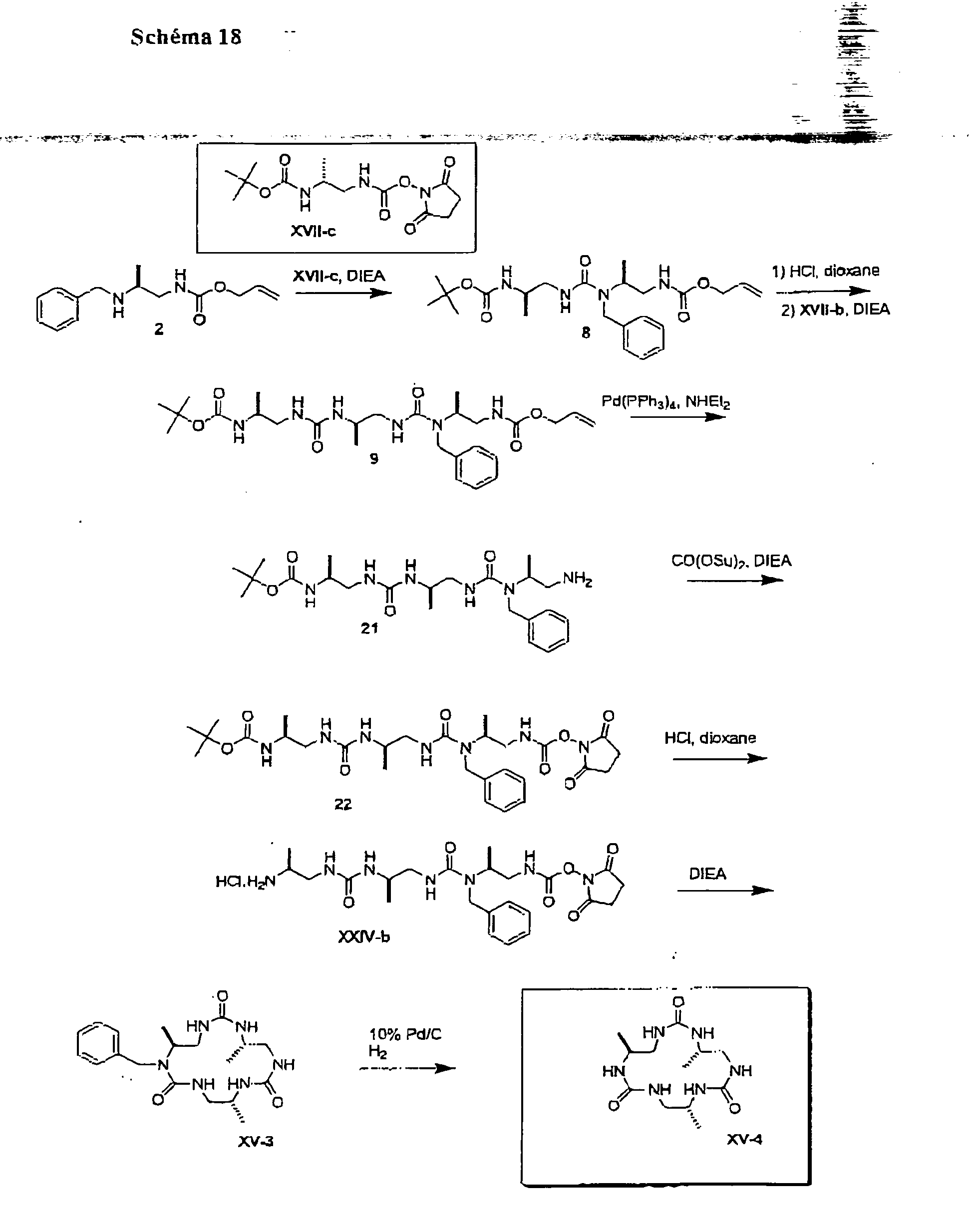

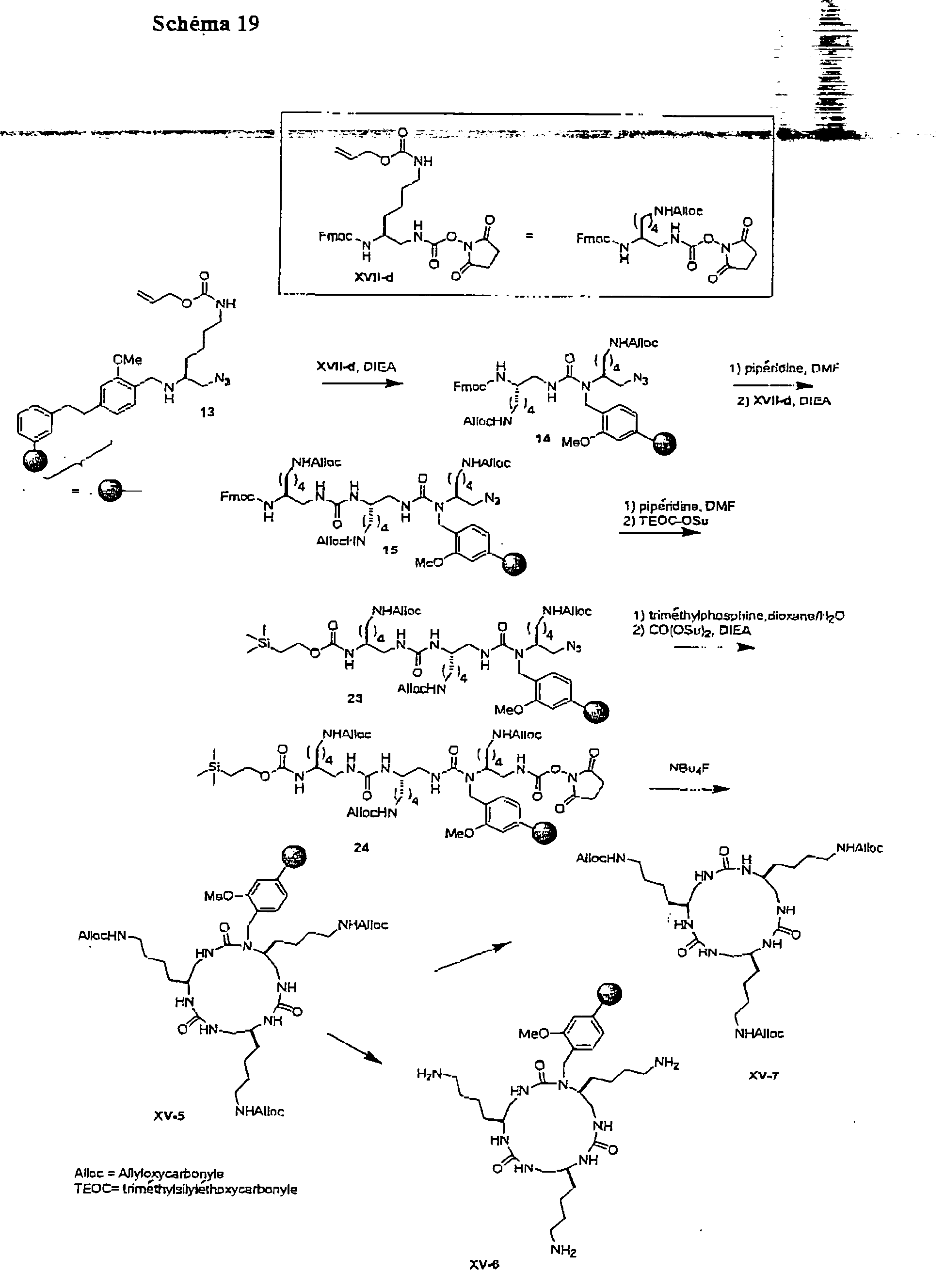

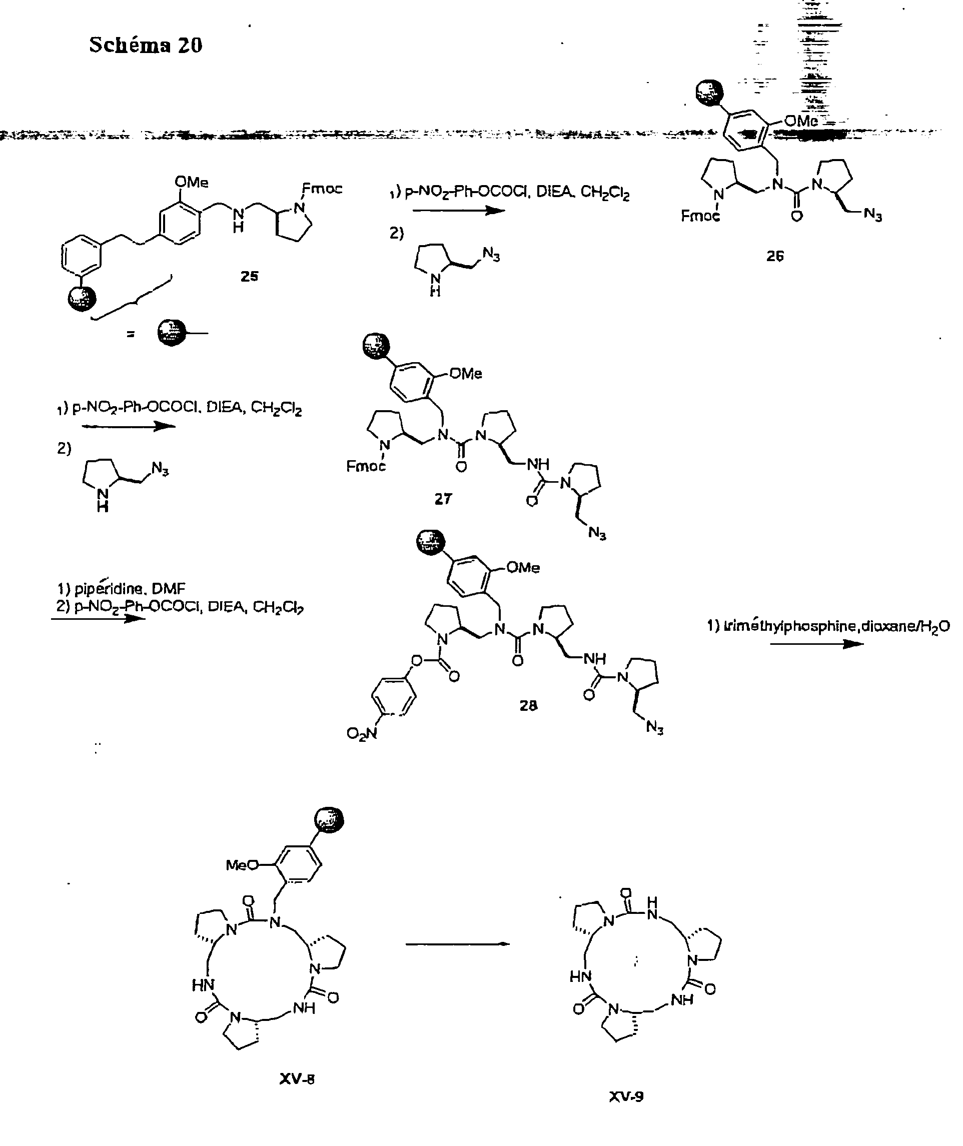

### Exemple 8

### Réactions de cyclisations intramoléculaires.

### 1. Importance de la géométrie de la liaison amide peptidique dans les dérivés d'acide carbamiques de formule (VIc).

Les expériences de cyclisation effectuées dans le cadre de la présente invention à partir de dérivés d'acide carbamique de type **(VIc)** (avec X= succinimide) indiquent que la géométrie de la liaison amide -CO-NR³- (liaison peptidique) joue un rôle important dans la nature des produits obtenus, et plus précisément dans la taille des cycles obtenus. La liaison peptidique se caractérise par sa géométrie qui a pu être établie à partir de données cristallographiques et RMN. La liaison peptidique -CO-NR³- peut être soit en configuration *trans*, soit en configuration *cis* (cf schéma 21). En général l'équilibre est fortement déplacé vers la forme *trans*. En présence de proline ou en cas de N-substitution, la forme *cis* peut devenir aussi importante que la forme *trans*.

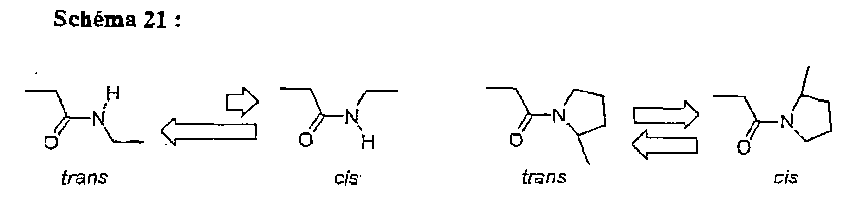

Les composés **(VIc)** pour lesquels R³ n'est pas un hydrogène, par exemple ceux pour lesquels R³ est un méthyle (cf composé (**VIc-6**) du schéma 8) ou ceux pour lesquels R³ forme avec R⁴ un cycle de proline à 5 atomes (cf composé (**VIc-5**) du schéma 7) cycliscnt de préférence sans réaction intermoléculaire avant cyclisation, pour donner uniquement le cycle à 7 atomes (monomère cyclique) correspondant de type **(Ia).** Ceci est très certainement dû à la préférence *cis* de la liaison amide dans ce type de composé. En effet dans ces cycles très contraints, la liaison amide est de géométrie *cis,* et cette géométrie doit être majoritaire afin de permettre la formation du cycle à 7 atomes.

La cyclisation des composés (**VIc**) pour lesquels R³ est un hydrogène ne conduit pas à l'obtention du monomère cyclique. Ceci s'explique par la préférence *trans* de la liaison amide -CO-NR³- dans ce type de précurseurs. La cyclisation intervient après une ou plusieurs réactions de couplage intermoléculaire, ce qui conduit à l'obtention d'oligomères cycliques de taille variable. Ainsi la cyclisation du composé **(XIXa)** (schéma 6) conduit préférentiellement à l'obtention du dimère cyclique **(IIIf-4)** (70%). Toutefois l'étude par spectrométrie de masse du brut réactionnel permet d'identifier les macrocycles de plus grande taille (trimère, tétramère, pentamère) suivant une distribution gaussienne.

### 2. Conditions préférées de cyclisation dans le cas des dérivés carbamate de succinimidyle de type (VIc).

L'importance d'un certain nombre de paramètres (ordre d'addition des réactifs, dilution, température), a pu être évaluée au cours de la cyclisation des dérivés **(XIXa), (XIXb)** et **(XIXc)** (cf. schémas 6, 7 et 8).

### • Ordre d'addition des réactifs

En général le dérivé d'acide carbamique contenant une fonction amine non protégée **((VIc-4), (VIC-5)** ou **(VIC-6))** solubilisé dans le solvant de réaction (solvant utilisé pour effectuer la cyclisation) (McCN par exemple) est additionné en goutte à goutte à une solution contenant une base et le solvant de réaction (MeCN par exemple).

Il est également possible d'inverser cet ordre en additionnant en goutte à goutte la solution contenant une base et le solvant de réaction à la solution contenant le dérivé à cycliser **((VIc-4), (VIc-5)** ou **(VIc-6))** et le solvant de réaction.

On obtient ainsi un mélange réactionnel contenant respectivement les composés urées cycliques (**IIIf-4**), (**Ii**) et (**Ij**).

### • Conditions de dilution

En ce qui concerne la dilution, la concentration du dérivé d'acide carbamique contenant une fonction amine non protégée ((**VIc4**), (**VIc5**) ou (**VIc-6**)) dans le solvant de réaction (solvant utilisé pour la cyclisation) n'a pas d'influence sur la cyclisation des dérivés **(XIXb)** et **(XIXc).** Dans ces exemples, les concentrations utilisées varient d'environ 1 M à environ 10⁻³ M.

Par contre, dans le cas de la cyclisation du composé **(XIXa),** la dilution a un effet sur la nature des composés cycliques obtenus. Lorsque la réaction est effectuée dans une solution diluée (contenant un dérivé d'acide carbamique contenant une fonction amine non protégée **(VIc4), (VIc5)** ou **(VIc-6)** dans le solvant de réaction) dont la concentration varie d'environ 10⁻⁵ M à 10⁻³ M, le produit majoritaire obtenu est le dimère cyclique **(IIIf-4)** (70%). Le trimère cyclique représente moins de 15%, et le tétramère cyclique moins de 5%. Par contre, lorsque la réaction est effectuée à concentration plus élevée (supérieure à 10⁻³ M), les réactions intermoléculaires sont nettement favorisées, et on obtient des cycles de plus grande taille (allant jusqu'au pentamère cyclique) dans des proportions plus élevées, au détriment du dimère cyclique.

### • Conditions de température

La température n'a qu'une faible incidence sur la cyclisation des produits **(XIXb)** et **(XIXc).** Ainsi, la réaction de cyclisation des dérivés **(XIXb)** et **(XIXc)** conduit respectivement aux dérivés **(Ii)** et **(Ij)** dans des rendements similaires, que la réaction ait lieu à 20°C, 0°C ou -10°C.

Par contre, le rendement de la cyclisation du composé **(XIXa),** conduisant au composé **(IIIf-4),** semble plus sensible à la température, le meilleur rendement étant obtenu lorsque la réaction est effectuée entre 0°C et 20°C. Pour des températures supérieures à 40°C, la réaction conduit à une augmentation notable de la formation des cycles de taille supérieure (augmentation de la réaction d'oligomérisation) alors qu'à une température de -20°C la réactivité de **(XIXa)** est très faible et ne conduit pas à l'obtention du composé **(IIIf-4).**

## Revendications

1. Composés urées cycliques comportant un cycle ayant au moins 8 atomes et comprenant au moins deux fonctions urées espacées l'une de l'autre par 2 atomes de carbones, répondant aux formules (XV) ou (XVIa), 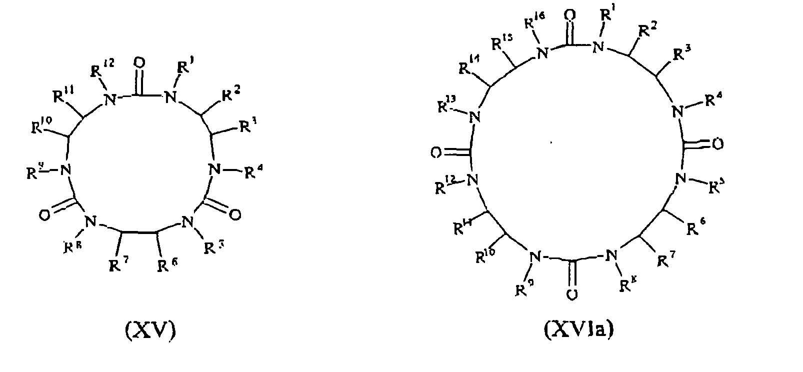 dans lesquelles les substituants R¹, R², R³, R⁴, R⁵, R⁶, R⁷, R⁸, R⁹, R¹⁰, R¹¹, R¹², R¹³, R¹⁴, R¹⁵ et R¹⁶ représentent :
- soit un atome d'hydrogène,
- soit la chaîne latérale protégée ou non d'un acide aminé choisi parmi les acides aminés naturels ou non naturels,
les groupes R¹, R², R⁵, R⁶, R⁹, R¹⁰, R¹³ et R¹⁴ pouvant également former les cyclisations intramoléculaires suivantes :
1/ cyclisation entre R¹ et R² et/ou,
2/ cyclisation entre R⁵ et R⁶ et/ou,
3/ cyclisation entre R⁹ et R¹⁰ et/ou,
4/ cyclisation entre R¹³ et R¹⁴,
et notamment dans lesquelles :
* R¹, R³, R⁴, R⁵, R⁷, R⁸, R⁹, R¹¹, R¹², R¹³, R¹⁵ et R¹⁶ représentent un atome d'hydrogène, et
* R², R⁶, R¹⁰ et R¹⁴ représentent un groupe choisi parmi les groupes méthyle, isopropyle, isobutyle, sec-butyle, benzyle, acétate d'alkyle et hydroxybenzyle (ortho, méta ou para),
sous réserve que les composés de formule (XV) ou (XVIa) soient différents des composés suivants de formules (VIII bis/1), (VIII bis/2), (VIII bis/3) et (VIII bis/4)

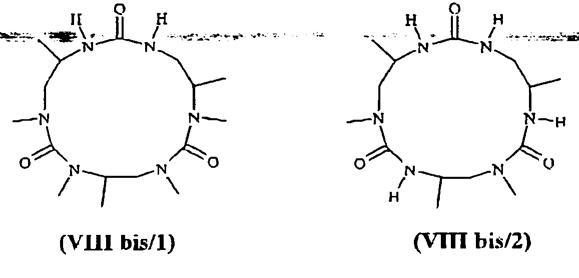

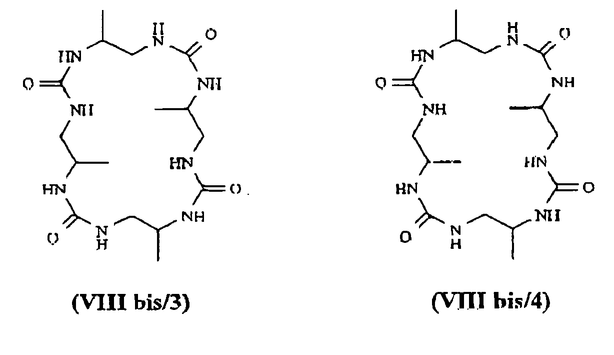

2. Composés selon la revendication 1, répondant aux formules suivantes : 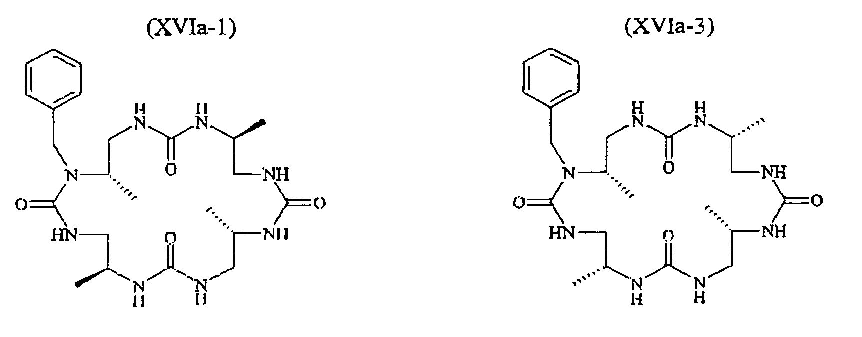 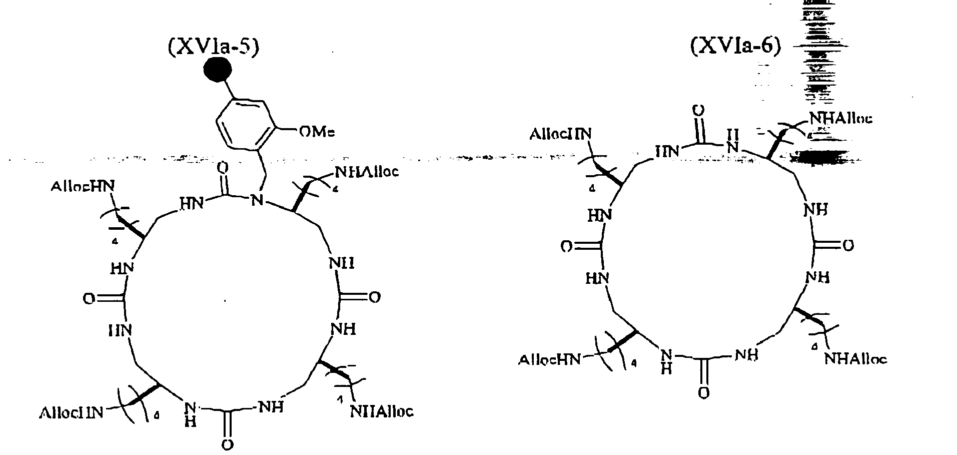 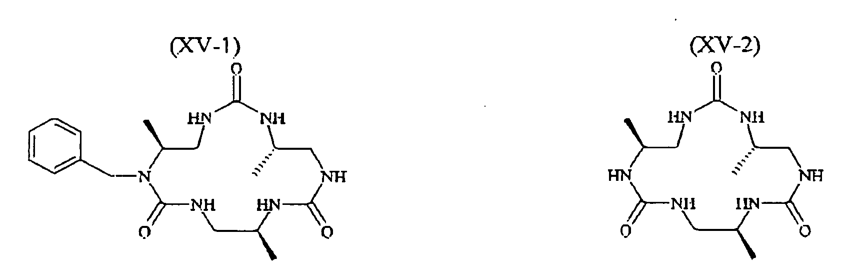 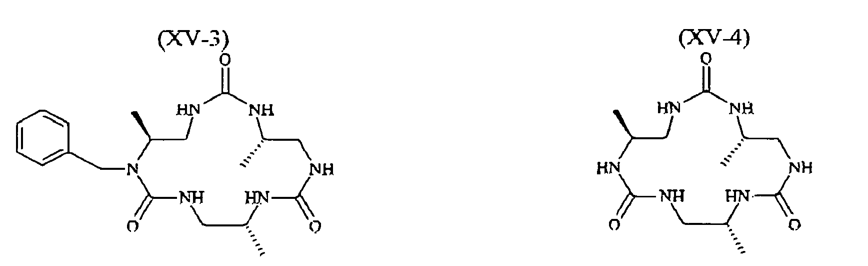 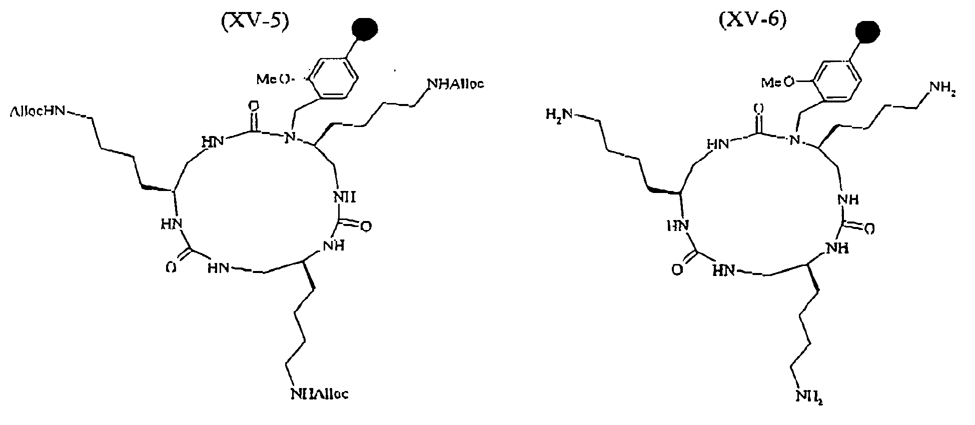 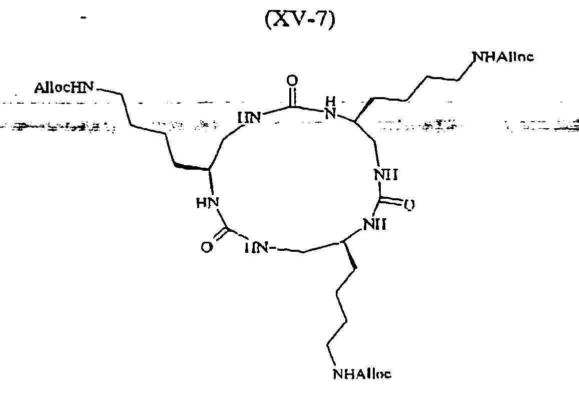 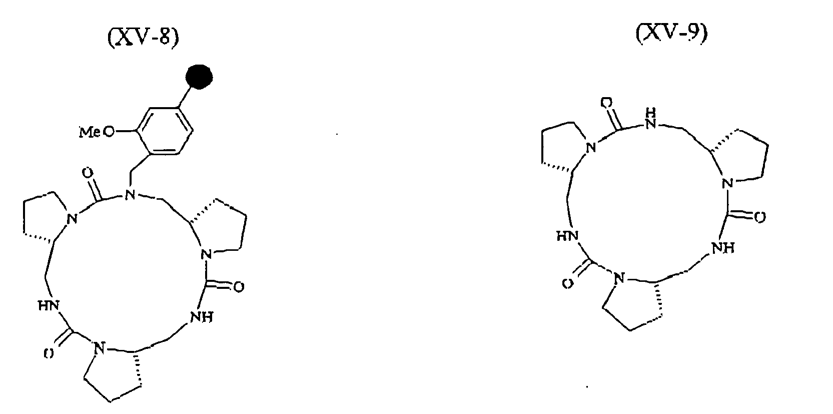 dans lesquelles Alloc représente un groupe allyloxycarbonyle et

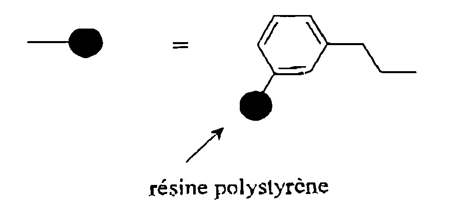

3. Procédé de préparation de composés urées cycliques répondant aux formules (XV) et (XVIa) telles que définies dans la revendication 1 ou 2, à partir d'au moins un dérivé activé d'acide carbamique contenant une fonction amine primaire ou secondaire non protégée, comprenant :
- une étape d'obtention d'au moins un dérivé activé d'acide carbamique contenant une fonction amine primaire ou secondaire non protégée, à partir d'au moins un dérivé activé stable d'acide carbamique contenant une fonction amine protégée par un groupe protecteur, par libération sélective de ladite fonction amine protégée dudit ou desdits dérivés activé(s) stable(s) d'acide carbamique, par clivage ou transformation dudit groupe protecteur,
- une étape de cyclisation par réaction entre la fonction aminé primaire ou secondaire non protégée d'au moins un dérivé activé obtenu à l'issue de l'étape de libération sélective et la fonction acide carbamique du ou des dérivés,
lesdits dérivés activés d'acide carbamique contenant une fonction aminé primaire ou secondaire répondant aux formules (XXIV) et (XXV) suivantes : 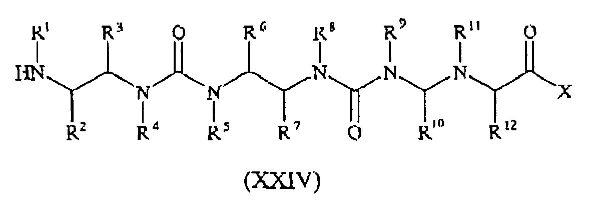 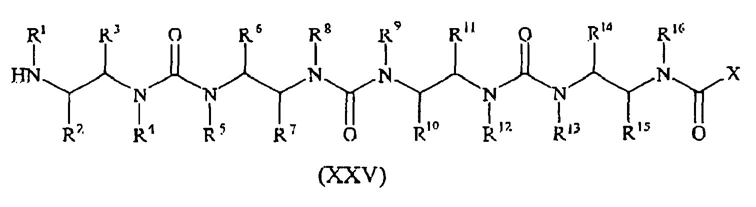 dans lesquelles
les groupes R¹, R², R³, R⁴, R⁵, R⁶, R⁷, R⁸, R⁹, R¹⁰, R¹¹, R¹², R¹³, R¹⁴, R¹⁵ et R¹⁶ sont tels que définis dans la revendication 1,
le groupe X représente un groupe conférant au dérivé une structure de dérivé activé d'acide carbamique, lequel groupe X étant issu d'un composé choisi notamment parmi les phénols, éventuellement substitués par au moins un groupe nitro ou au moins un halogène, ou les dérivés d'hydroxylamine, ou les dérivés d'alcools benzyliques greffes sur support solide et plus particulièrement choisi parmi les composés suivants : N-hydroxysuccinimide, phénol, pentafluorophénol, pentachlorophénol, p-nitrophénol, 2,4-dinitrophénol, 2,4,5-trichlorophénol, 2,4-dichloro-6-nitrophénol, hydroxy-1,2,3-benzotriazole, 1-oxo-2-hydroxydihydrobenzotriazine (HODhbt), 7-aza-1-hydroxy-benzotriazole (HOAt), 4-aza-1-hydroxybenzotriazole (4-HOAt), imidazole, tétrazole, résine WANG,
lesdits dérivés d'acide carbamique pouvant également être sous forme protonée.

4. Procédé de préparation selon la revendication 3, de composés de formule (XV), dans laquelle :
- les substituants R², R⁶ et R¹⁰ sont choisis parmi :
* un atome d'hydrogène
* une chaîne alkyle C1-C7, linéaire ou ramifiée, substituée ou non par :
i) une fonction amine protégée ou non,
ii) une fonction acide protégée ou non,
iii) une fonction alcool protégée ou non,
iv) un groupement aryle ou hétéroaryle,
* la chaîne latérale protégée ou non d'un acide aminé choisi parmi les acides aminés naturels ou non naturels,
- les substituants R¹, R³, R⁴, R⁵, R⁷, R⁸, R⁹, R¹¹ et R¹² représentent un atome d'hydrogène,
à partir de dérivés activés d'acide carbamique de formule (XXIV), telle que définie dans la revendication 3, dans laquelle :
i) les substituants R², R⁶ et R¹⁰ ont les significations mentionnées ci-dessus et les substituants R¹, R³, R⁴, R⁵, R⁷, R⁸, R¹¹ et R¹² représentent un atome d'hydrogène, ou
- les substituants R³, R⁷ et R¹¹ ont les significations mentionnées précédemment pour R², R⁶ et R¹⁰, et les substituants R¹, R², R⁴, R⁵, R⁶, R⁸, R¹⁰ et R¹² représentent un atome d'hydrogène,
ii) le groupement R⁹ est un groupe arylalkyle ou hétéroarylalkyle, qui peut être relié à un support solide,
ct permettant la formation, avant l'étape finale de déprotection, d'un intermédiaire de synthèse ayant la formule (XV), telle que mentionnée ci-dessus, dans laquelle les différents substituants R¹ à R¹² ont les mêmes significations que mentionnées ci-dessus pour le composé de formule (XXIV).
